# EUROPEAN PATENT SPECIFICATION

(11) **EP 3 972 471 B1**
(45) Date of publication and mention of the grant of the patent: **03.07.2024**
(21) Application number: 19744878.0
(22) Date of filing: 21.05.2019
(51) Int. Cl.: A47L 11/20, A47L 11/206

(54) **DEVICE FOR CLEANING WALKABLE SURFACES**
VORRICHTUNG ZUR REINIGUNG VON BEGEHBAREN FLÄCHEN
DISPOSITIF POUR LE NETTOYAGE DE SURFACES PRATICABLES

(43) Date of publication of application: 30.03.2022
(73) Proprietor: Technological Systems by Moro S.r.l., 33080 Fiume Veneto (PN) (IT)
(72) Inventor: MORO, Gianpietro, 33080 San Quirino (PN) (IT); MORO, Andrea, 33170 Pordenone (IT)
(74) Representative: Di Gennaro, Sergio
(86) International application number: PCT/IT2019/000039
(87) International publication number: WO 2020/234904

(56) References cited:
- GB-A- 201 604
- US-A- 3 186 022
- US-B1- 6 421 868

## Description

The invention relates to a device for cleaning walkable surfaces, adapted to move forward automatically and to be steered manually by an operator for cleaning walkable surfaces, as for example floors, roads, pavements, etc., particularly where there are present tight spaces for operating such a device or spaces that are difficult to be reached.

To date we know different types of machines for cleaning floors by means of motorized rotating brushes, which are also wet by means of a relative delivery system for liquids.

Particularly, in the technical field of the present invention, it is known a first type of floor scrubber machine constituted by a lower support frame leant on wheels and one or more motorized brushes with vertical rotation axis or slightly inclined, adapted to rub the floors to be cleaned and to move forward automatically the cleaning machine thanks to the friction with the floor, without the operator must push it or without the use of motorized wheels.

Such a frame substantially supports on its upper part the unit motor-battery for actuating such cleaning brushes, the tanks for the clean water, for the possible detergent, and for the dirty water, sucked by an electrical suction system, which is placed on the upper part of said frame too and is associated with an element, which is lengthened horizontally and made of rubber, positioned lowerly in the back part of said support frame, and adapted to collect the dirty water resulted from the brushing action of the floor by the wetted rotating brushes.

A fixed guiding body is also secured on the support frame, which is lengthened upward and ending with a handle associated with a console equipped with commands adapted to be grasped by the operator for guiding the cleaning machine and for commanding the electrical and electronic parts thereof.

The component elements supported on the upper part of the support frame and to the lower part of the guiding means are covered by a shaped casing.

Such a type of cleaning machine for floors, also if it is easily to be guided and commanded, is not adapted to clean floors and tight spaces or in spaces wherein objects are placed on the top and spaced away from same, as for example hanging furniture, tables, chairs, etc., because the machine has considerable dimensions.

In this technical field, it is also known a second type of machine for a wet cleaning of floors by means of rotating brushes, constituted by a base body connected, by means of articulated jointing element, to an upper lengthened guiding means.

Said base body comprises at its lower part one or more motorized rotating elements, as for example brushes, rollers with bristles, rollers with sponge, etc., with a rotation axis that is horizontal, vertical or inclined, adapted to scrub the floors for cleaning them.

An element, made of rubber, is positioned behind said motorized rotating elements, with respect to the direction of advancement of the machine, and is adapted to receive and to keep the dirty brushed from the floor, that is then sucked by means of a suction system and collected in a suitable tank.

It may be present also a wetting system of said motorized brushes or similar, for increasing the cleaning efficiency thereof.

On the guiding means, in addition to the handle for guiding the machine, there are present the command for setting the operational functions of the same machine.

For facilitating the operator, such rotating elements may move forward the machine during the brushing, thanks to the friction with the floor.

Various component elements, including the motors, the batteries, the tanks, the operation electrical and electronic systems, the elements of the wetting and suction systems are applied and arranged between the upper part of the base body and the guiding means, in particular with at least tank applied on this latter.

This second type of known cleaning machines use a articulated jointing element between the guiding means and the base body, in a manner to be able to bring the upper end portion of the same guiding means nearer to the floor, so that it is possible to clean portions of floors that are covered in a spaced manner by tables, chairs, hanging furniture, etc..

As articulated jointing elements there are used joints with one rotation axis, for example that allow a tilting movement of the guiding means, or joints with two rotation axis orthogonally to each other and placed on two different levels, as for example a universal joint, that allow to the guiding means to be bent with its upper end portion toward the floor in different direction, and by twisting the same guiding means, they allow that the base body may rotate toward right or left.

In this second type of cleaning machine, the lengthened guiding means may be inclined in a limited manner with respect to the base, up to it goes into contact with it, and this may be result a limitation for cleaning areas of the floor with objects positioned above it and spaced superiorly, and that have a considerable depth.

Furthermore, the base may not rotate on itself on a vertical axis without inclining on a horizontal axis, except with the guiding means positioned vertically, and that in narrow spaces, wherein it is necessary to incline the same guiding means, compels the operator to pull the cleaning machine in the reverse direction with respect to the one in which the motorized rotating elements create propulsion, therefore by tiring the operator and by straining the motors of said rotating elements. This type of cleaning machine is not provided with dampening means that damp collisions with external elements, places on the floor to be treated, thereby creating the risk of damages to the same machine.

By placing the tanks for clean water and/or dirty water on the guiding means, in addition to the weight of the liquid contained in the tanks, the water, passing from a tank to the other one, changes the weight distribution on the guiding means, thereby causing a change of the operator's posture. (insert page 3a which is numbered as page 1a). The object of the present invention is to delete the above said problems and limits, and additional advantages will be described following.

The prior document US3186022A discloses a surface treating apparatus comprising a downwardly opening base, rotatable surface treating means mounted within said base and projecting downwardly from the latter for contact with a surface to be treated, a motor-fan unit on said base having air inlet means at a side of said unit and air outlet means, said motor-fan unit being operative to drive said surface treating means and to produce a decreased pressure at said air inlet means for inducing an air flow into the latter, said base having a suction passage opening at the bottom of the base, connection means on said base located adjacent said side of the motor-fan unit and communicating with said suction passage, dust collecting means including a porous receptacle having a mouth engaged with said connection means and a cover removably mounted on said base to define a chamber enclosing said receptacle and communicating with said air inlet means of the motor-fan unit.

This aim and other ones are obtained according to the invention by means of the use of a device for cleaning walkable surfaces realized in a different manner with respect to the known solutions from the prior art, with the characteristics and operating as it will be described, by way of a not-limitative example only and with reference to the accompanying drawings, in which :
- Fig. 1 shows a front-side perspective view of a first example of the device for cleaning walkable surfaces in a starting rest position thereof, according to the invention ;
- Fig. 2 shows a back-side perspective view of the device of Fig. 1;
- Fig. 3 shows a back perspective view of the cleaning device of Fig. 1 in an operational position thereof, without some of component elements thereof for giving prominence additional internal component elements thereof;
- Fig. 4 shows a view from below of the cleaning device of fig. 1 ;

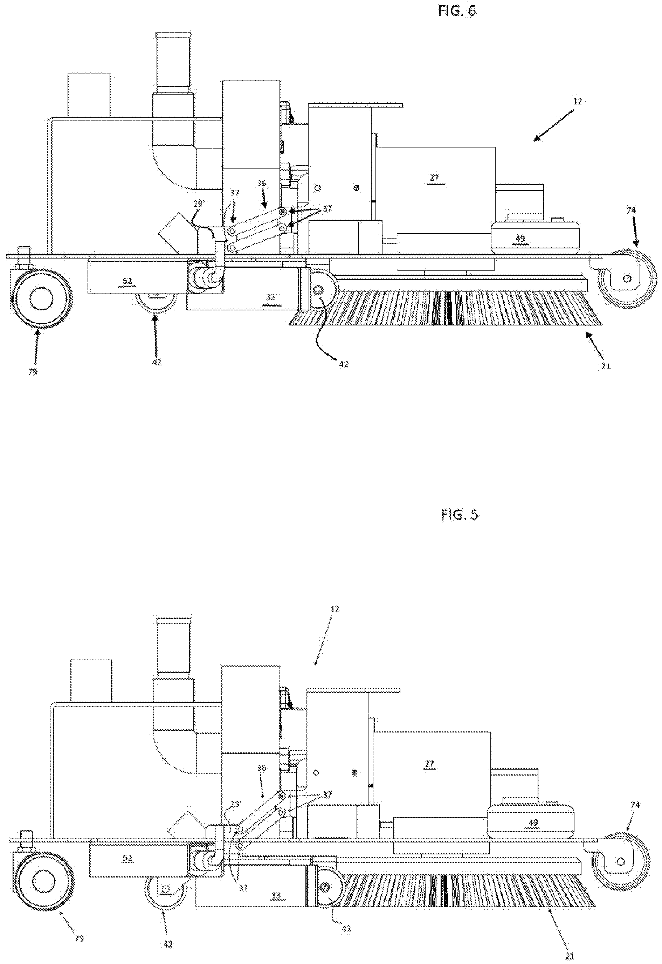

- Fig. 5 shows a side view of a group of elements that is shown in fig. 3, in a working position thereof;
- Fig. 6 shows a side view of the group of elements of Fig. 5, in a not-working position thereof;

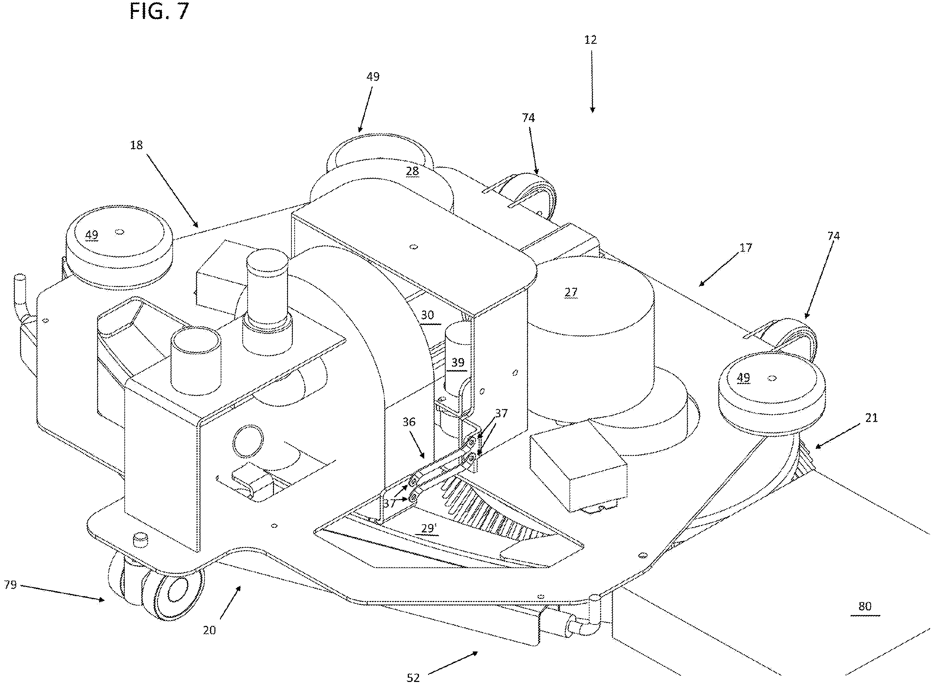

- Fig. 7 shows a back-side perspective view of the group of elements shown in Fig. 3, in an operational position thereof, and one external element;

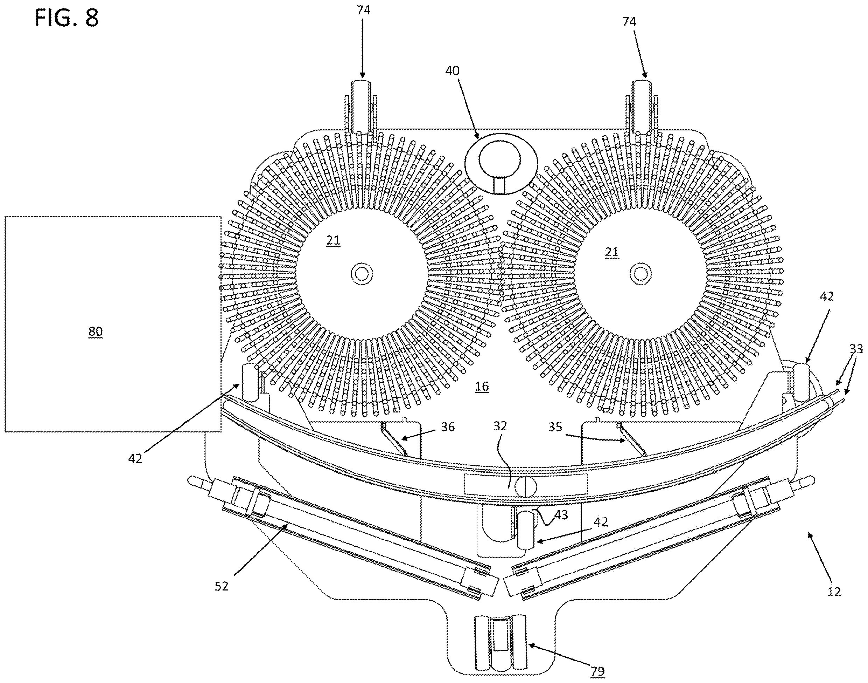

- Fig. 8 shows a view from below of fig. 7 ;
- Fig. 9 shows a front view of the cleaning device in an operational position thereof;
- Fig. 10 shows a back-side perspective view of the cleaning device shown in Fig. 9 ;
- Fig. 11 shows a side view of the cleaning device in an additional operational position thereof;
- Fig. 12 shows a back-side perspective view of the device of Fig. 11, together one external element;
- Fig. 13 shows a perspective view from the top of the cleaning device, in an additional operational position thereof;
- Fig. 14 shows a front-side view of the cleaning device, in an additional operational position thereof;
- Fig. 15 shows a back-side view of the cleaning device, in an additional operational position thereof;
- Fig. 16 shows a perspective view of a group of internal elements of the cleaning device with one element in a rest position thereof;
- Fig. 17 shows a perspective view of the group of internal elements of Fig. 16, with the above said element in a operational position thereof;
- Fig. 18 shows a side view of the cleaning device in a storage/packaging position thereof ;
- Fig. 19 shows a side perspective view of the group of elements of Fig. 18 ;
- Fig. 20 shows a side view of a second example of the cleaning device in a working position thereof, together one external object.

The invention relates to a device for cleaning walkable surfaces, adapted to move forward automatically and to be steered manually by an operator for cleaning walkable surfaces, as for example floors, roads, pavements, etc., particularly where there are present tight spaces for operating such a device or spaces that are difficult to be reached.

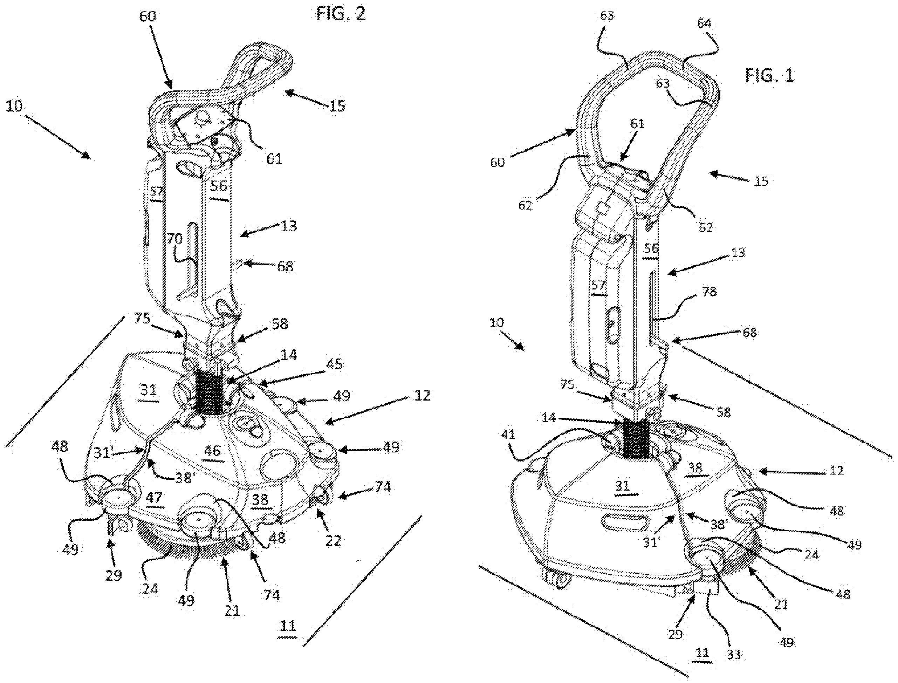

As it is visible particularly in Figs. 1-2, the device 10 is realized as scrubber-dryer machine for cleaning walkable surfaces 11, as for example floors, pavements, asphalted roads, etc., and is substantially constituted by a cleaning lower base body connected on its upper part to a steering means lengthened upward, by means of a movable jointing element 14, which is conformed in such a way that such a lower body 12 may move forward at least in one travel direction and that said steering means 13, from one starting position thereof perpendicular with respect to the base body 12, be inclinable in all direction without that the base body 12 being inclined, up to be able to touch the walkable surface 11 with its upper free end portion 15, and may also steer said lower body 12 apart from the inclination wherein it is, as it will be described.

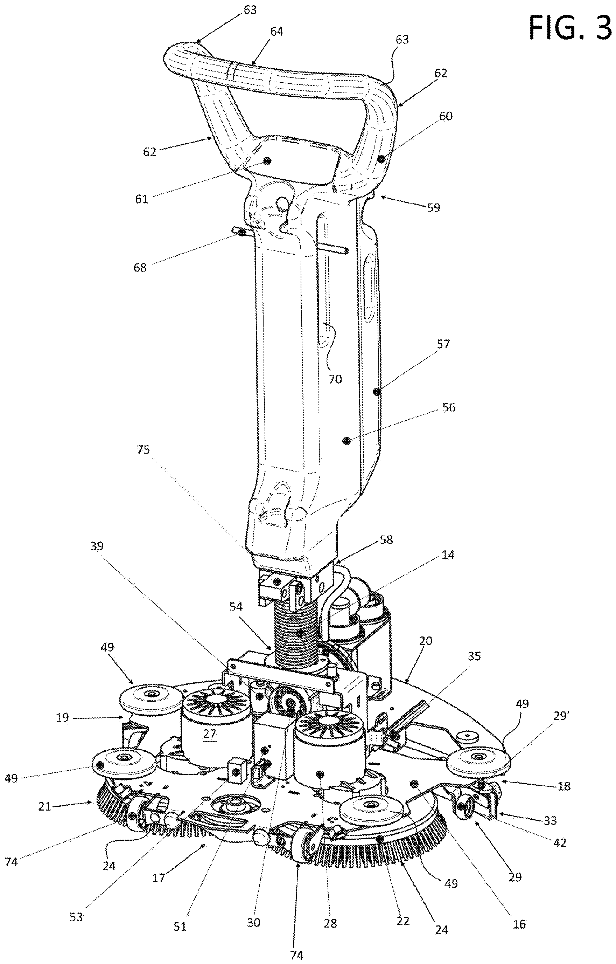

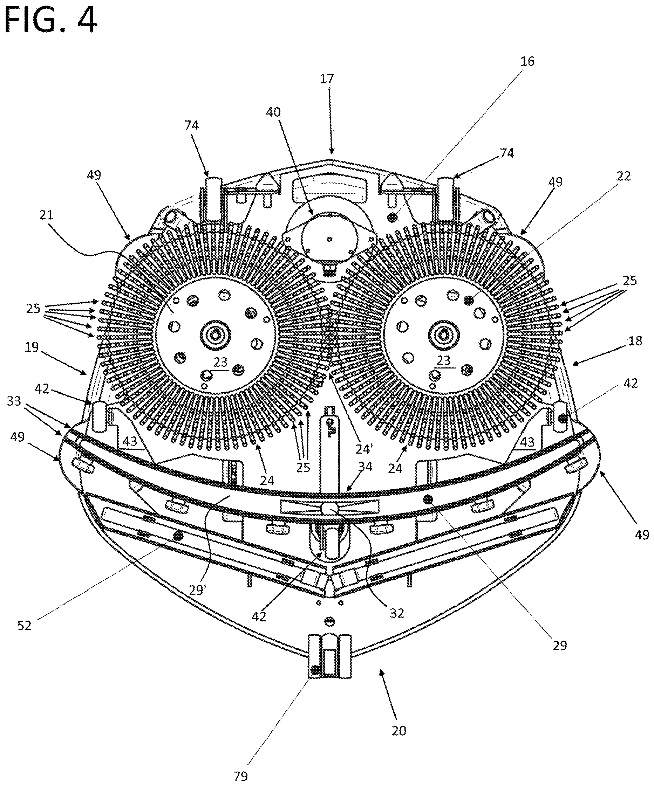

As it is visible particularly in figs. 3-4, said cleaning lower body 12 is constituted by a support frame 16 having a front outline 17, two side outlines 18, 19, and a back outline 20, and onto which there is fixed the movable jointing element 14, in such a way to be positioned in the barycenter of the same lower body 12.

At least a brushing means is housed lowerly to such a support frame 16, by means of bearings or axle boxes or equivalent means, in such a way to be rotatable along an axis slightly inclined with respect of the vertical of the device 10 leant on to a horizontal plane, thereby be able to brush the walkable surface 11 and removing the dirt present on this latter.

In the not-limitative examples described herein there are provided two brushing means 21 and 22, identical to each other, and each constituted by a raised cylindrical plate 23 provided with a plurality of bristles 24 arranged radially in the lower part of the same cylindrical plate 23 and directed outward with respect to this latter and downward.

Said two brushing means 21 and 22 are arranged side by side to each other in a transversal manner with respect the orientation of the lower body 12, in such a way that an internal portion 24' of the bristles 24 of each means 21, 22 is intersected with its own end portions 25, whereas one external portion of the bristles leans out laterally with its own end portions 25 with respect to the outline of the lower body 12, furthermore the rotation axis of both brushing means 21 and 22, with extension upward, is slightly inclined inward the lower body 12, with the same angle, in such a way that the bristles 24 of the internal portions 24' be more pressed against the walkable surface 11 to be cleaned.

Said brushing means 21 and 22 are actuated in rotation by means of respective electrical actuating means 27, 28, constituted as for example by electrical motors, which are arranged on the upper part of said frame 16, and above to the respective brushing means 21 and 22, that they have to actuate. Said brushing means 21 and 22 rotates in opposite directions to each other, in particular the internal portion 24' of the bristles 24 move backward with respect to the direction of advancement of the device 10, in such a way to convey the brushed dirty in a central and back position with respect to themselves, and in such a way that, the friction made by the rotation of the brushing means 21 and 22, with an inclined rotation axis, between the internal portions 24' of the bristles 24 and the walkable surface 11 made in turn a traction such to allow to the device 10 to move forward automatically in a rectilinear way when the brushing means 21 and 22 are actuated in rotation.

At least a system for collecting the dirty conveyed by the internal portion 14' is placed on the back and spaced away from said brushing means 21 and 22, which system is constituted by at least a restraining means 29 for the dirty, adapted to restrain the conveyed dirty, associated to at least a suction means 30, adapted to suck the restrained dirty, and at least a collection means 31, adapted to contain the sucked dirty.

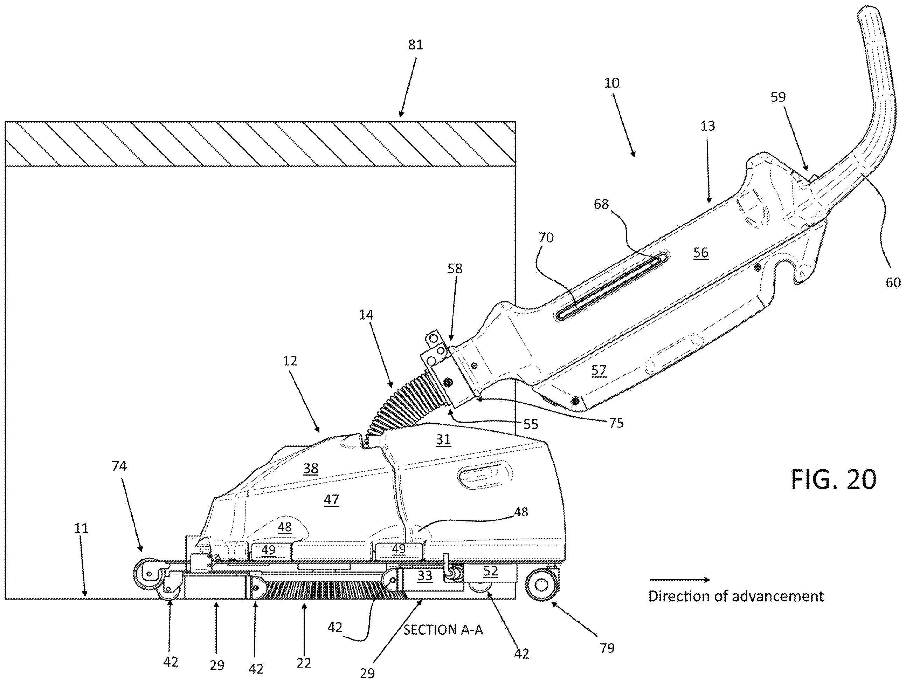

In the case it is provided for a manually or automatic inversion of the rotation direction of the brushing means 21 and 22, at least a second system for collecting the dirty constituted by at least a restraining means 29 for the dirty, at least a suction means 30, and at least a collection means 31, is placed on the front part and spaced away with respect to the same brushing means, in such a way that in the case of the inversion of the rotation direction of these latter, it is always at the back with respect to them, such a second example of device 10 is represented in Fig. 20.

Said restraining means 29 for the dirty is constituted by a lengthened semi-circular support 29' provided with at least a vertical through hole 32 and which supports on its lower edge at least a lengthened element 33, made of a semi-rigid material, as for example rubber, said support 29' being positioned transversally with respect to the orientation of the device 10, with its concave part 34 turned toward the two brushing means 21 and 22, and extending between the two side outlines 18, 19, whereas the lengthened element 33 being extended beyond these latter.

Said support 29' is connected to the lower part of the frame 16 by means of at least two pair of levers 35, 36, pivoted at their end portions 37 parallel to each other, and arranged longitudinally with respect to the orientation of the device 10, thereby creating a pantograph linkage system, which levers are made of elastic metal, preferably of harmonic steel, and are adapted to rotate on their end portions 37 according the vertical movement caused by the restraining means 29 that follows the conformation of the walkable surface 11, thereby keeping itself always parallel to this latter, or it may be lifted or lowered manually or automatically when the operator considers it necessary, that is when it is foreseen the inversion of the rotation direction of the brushing means 21 and 22, and said collection system of the dirty is on the front with respect to the direction of advancement of the device 10 in an operational state thereof, furthermore, such levers are structured and adapted to be bent transversally when the restraining means 29 bumps laterally against an external obstacle 80, for then returning automatically in the original position thereof after having passed such an obstacle.

Such a restraining means 29 may be lifted by rotating the levers 35, 36 in a manual way or by means of one electrical actuator means 51, placed on the upper part of the frame 16, and that operates in the manner that will be described further, said restraining means 29 being kept lifted by means of quick fixing means or magnetic means or electro-magnetic means, or equivalent.

Said restraining means 29 may be provided with two support wheels 42, placed laterally and/or centrally with respect to the lengthened support 29' and are fixed by means of metallic brackets 43. Said suction means 30 is of the electrical type, and is arranged on the upper part of said frame 16 and above said restraining means 29, and with its inlet mouth is connected in a communicating way to said vertical through hole 32, in such a way that when it is actuated it may suck through it the dirty restrained by the same restraining means 29.

The outlet mouth of such a suction means 30 is connected in a communicating manner to said collection means 31, in such a way that the sucked dirty may be conveyed and collected inside it. Said collection means 31 is constituted by a first tank arranged in a removable manner above the back half portion of said frame 16 and the components placed in this portion of the frame, and has a base that follows the outline and the dimensions of the portion of frame on which is leant and a height that doesn't interfere with the jointing element 14.

Said brushing means 21, 22 are associated with a wetting system adapted to force the washing liquid on to the same means, for making most efficient the cleaning of the walkable surface 11, for then be mixed to the brushed material to be removed and conveyed by the bristles 24 toward said dirty collection system.

Said wetting system is constituted by one containment means 38, adapted to contain the cleaning liquid, one electrical pumping means 39, adapted to draw said cleaning liquid, and delivery means 40 for delivering such a liquid on the brushing means 21, 22.

Said electrical pumping means 39 is constituted for example by an electrical pump and is placed on the frame 16, in the central area of this latter, whereas said containment means 38 is constituted nu a second tank arranged in a removable manner above the front half portion of said frame 16 and the components placed in this portion of frame, and has a base that follows the outline and dimensions of the portion of frame on which it is leant and an height that doesn't interfere with the jointing element 14.

The back wall 38' od said second tank 38 and the front wall 31' of said first tank 31 are almost vertical and coupled to each other thereby forming a unique body 45, constituted by two above said tanks 31, 38, and having an upper surface 46 almost continuous and inclined frontally downward and side walls 47 inclined inward and upward, and finally having a vertical hole 41, preferably with a cylindrical cross-section, in correspondence of the jointing element 14, in such a way that this latter be inserted therein and be free to carry out its function, cylindrical hole 41 obtained by means of two vertical semi-cylindrical grooves identical and specular to each other, and provided on to two said vertical walls 31', 38' coupled to each other, respectively.

Such a body 45, along its lower base outline, is also provided with a plurality of shape recesses 48 identical and equidistant to each other, for the aim that will be described further.

Said delivery means 40 are constituted by one or more nozzles positioned in the lower part of the frame 16 and connected in a communicating manner to said pumping means 39 and turned on to the brushes 21, 22, in such a way that these latter may be wetted with the cleaning liquid drawn from the second tank 38.

Above said frame 16, a respective impact resistant means 49 is housed in correspondence of each recesses 48 of the body 45 and is protruded partially outward the body 45 with its side edge, and is adapted to absorb eventual impacts with external objects with respect the device 10, such an impact resistant means 49 is constituted for example by a roller pivoted with a vertical rotation axis, free in rotation, and made of semi-rigid elastic material, as for example rubber.

At least one electrical sanitation means 52 is fixed to the lower part of the frame 16, at the back with respect to the direction of advancement of said device 10 and to said restraining means 29, such a sanitation means 52 being constituted as for example by one or more UV lamps and being adapted to complete the sanitation of the walkable surface 11 just treated.

At least a pivoting wheel 79 is positioned in the lower part of the frame 16, at the back with respect to said electrical sanitation means 52, and is adapted to support the back part of the lower body 12, by avoiding that this latter is inclined backward.

The device 10 may be equipped with at least a detecting means 53, constituted for example by a sensor of the gyroscope type or inertial type, positioned on the frame 16 too, and adapted to detect the direction of advancement of the same device 10, that is if the operator is pushing or pulling the same device 10 for inverting the direction of advancement thereof, therefore the brushing means 21 and 22 may eventually invert the own direction of rotation and that the possible restraining means 29 positioned on the front with respect to the brushing means 21 and 22 with respect to the advancement direction of the device 10 be lifted up from the walkable surface 11, while the possible restraining means 29 positioned on the back of the brushing means 21 and 22 with respect to the advancement direction of the device 10 be lowered and put into contact with the walkable surface 11, such an operation of lifting and lowering of the possible restraining means 29 is obtained, as above described, by means the rotation of the levers 35, 36, both in a manual manner and thanks to the actuator means 51, that may be associated to the sensor means 52 by working automatically or being commanded directly by the operator by means of suitable commands.

Said movable jointing element 14, fixed vertically on the frame 16, in the barycenter point of the lower body 12, is adapted to connect the cleaning lower body 12 and the upper steering means 13 in a constraint way in rotation with respect to the vertical axis of the connecting point between the jointing element 14 and the lower body 12 and in a non-constraint way in inclination of the steering means 13 with respect to the lower body 12.

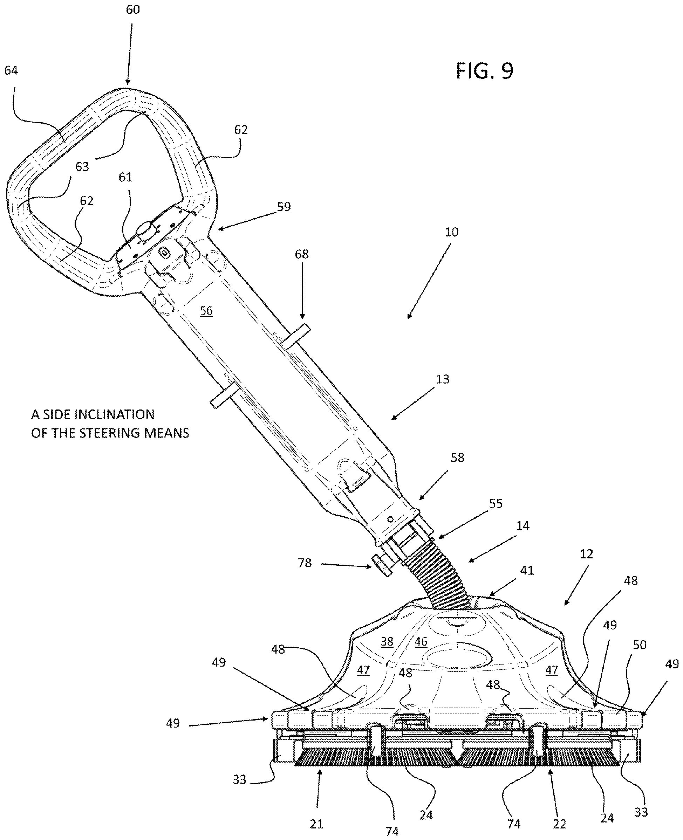

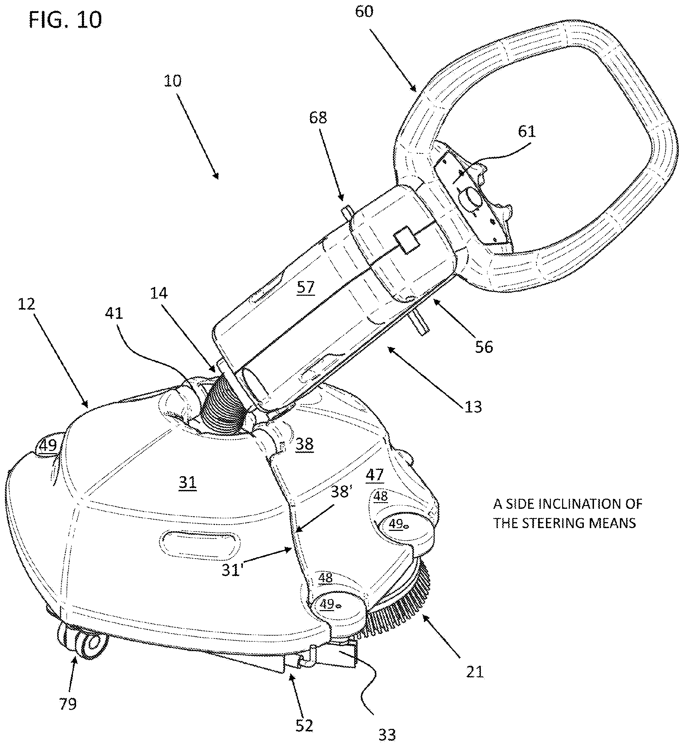

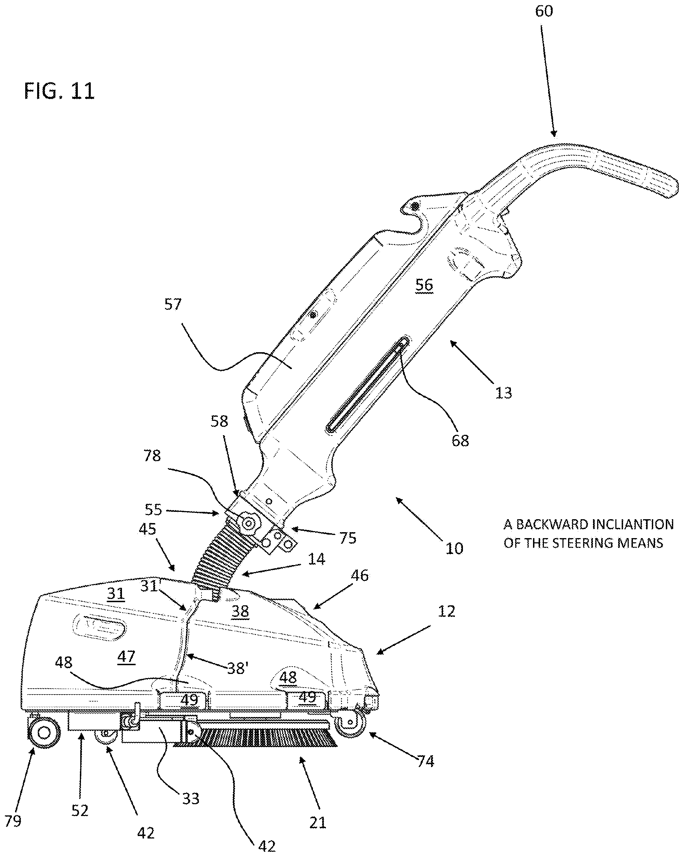

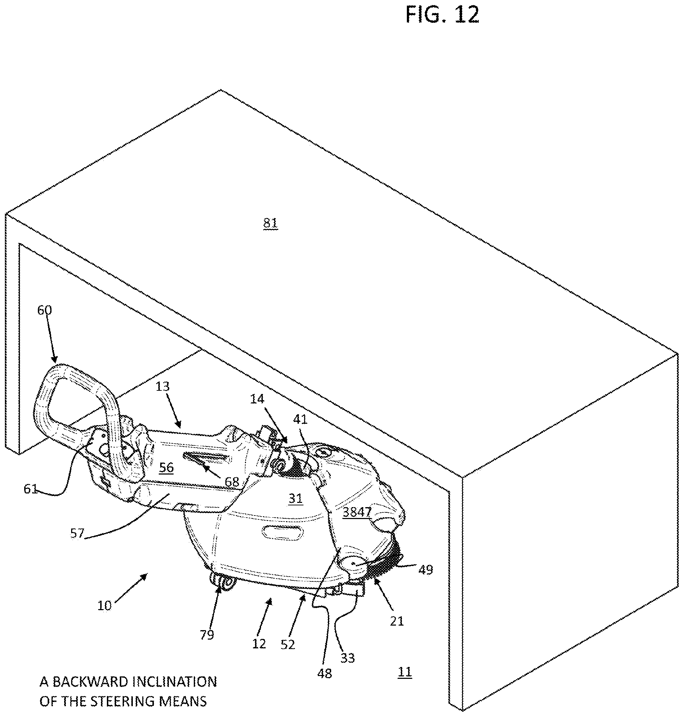

Such a jointing element 14 is constituted by at least a lengthened elastic and internally hollow push element 14 that in a rest position thereof has a rectilinear upward extension, and is constrained with its lower end portion 54 to the frame 16 and with its upper end portion 55 to the steering means 13, such a lengthened elastic element may be taken after a curvilinear extension by inclining the steering means 13, that is by moving forcefully its upper end portion 55 downward and externally and in any direction with respect to the vertical axis of the same elastic element in the rest position thereof, being able in this position with curvilinear extension to be rotate along the vertical axis of the same elastic element in the rest position thereof, without that the lower body 12 be interested by such a rotation (See Figs. 9-12, 20).

The elastic element 14 may be curved downward up to the steering means 13 doesn't touch the walkable surface 11, so also with inclinations up to at least 90° with respect the start position thereof, allowing to the operator to insert the apparatus 10 in to space where there are present external objects 81 at the top, as for example small tables, hanging furniture, etc., without that the steering means 13 finds interferences.

By decreasing the downward push force of the upper end portion 55, the elastic element return toward the rest position, up to reach it with the removal of such a downward push force, therefore by reducing also the weight load on the operator's arms.

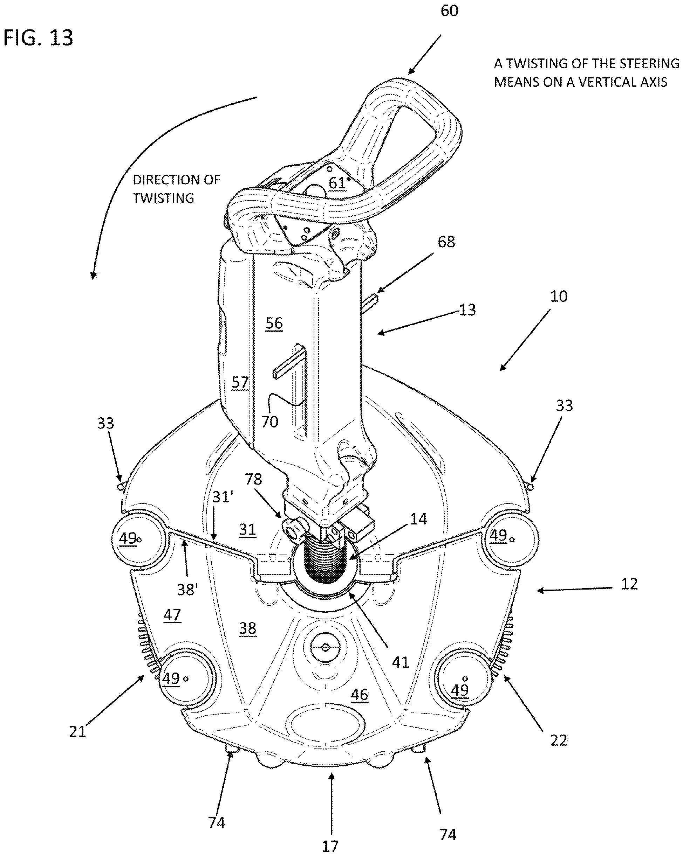

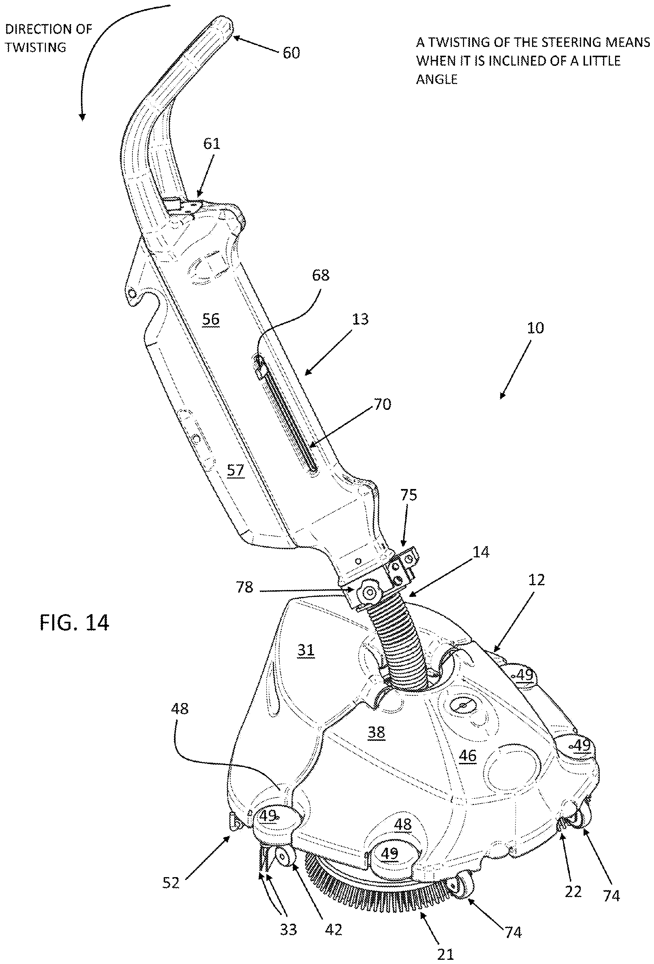

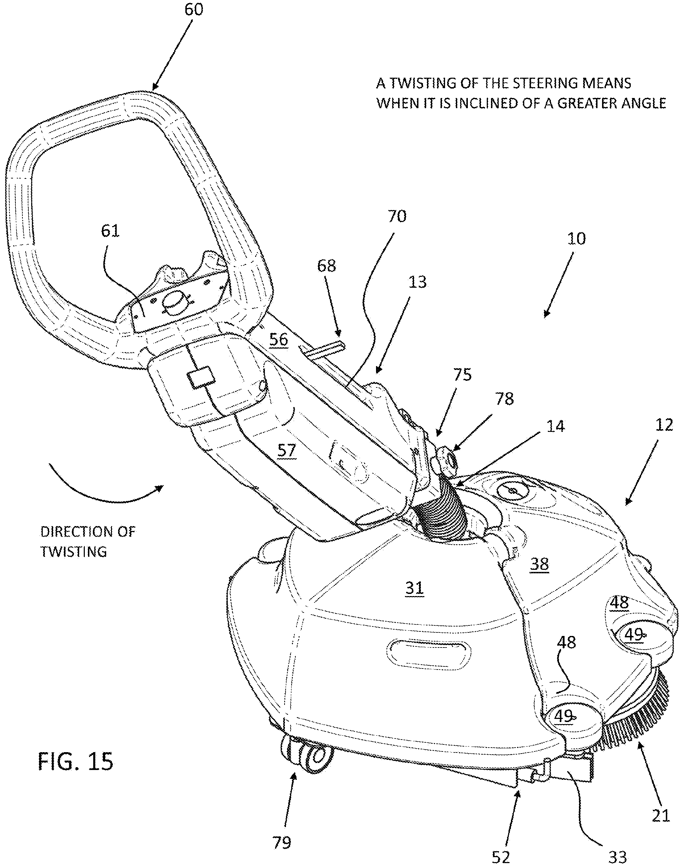

By applying a twisting moment to the upper end portion 55 of the lengthened elastic element 14, this latter twist along the own axis, both that it is in the position with rectilinear extension thereof and that it is in a position with a curvilinear extension thereof, by creating a twisting moment in the connecting point between its lower end portion 54 and the frame 16, by allowing to the lower body 12 to rotate on its perpendicular axis extent upward of the angle desired by the operator, without that it be inclined, so by using a limited space for change direction, and so it is advantageous for the cleaning in tight spaces. Furthermore, by rotating the lower body 12 on itself, this latter from a spaced position from the operator, it returns close to the same operator automatically, always in the starting direction of advancement (See Figs. 13-15).

Such a lengthened elastic element 14 is constituted by a sprung element, as for example:
- one spring, made of metal or polymer (shown in the attached drawings) ;
- at least two springs inserted or co-moulded concentrically one inside the other, made of metal and/or polymer ;
- one metal bellow, as for example made of steel, with a core made of elastic and semi-rigid material, as for example rubber;
- a pipe made of elastic and semi-rigid material, as for example rubber, provided with an internal reinforcement made of reinforcing fibers woven to each other, as for example wire made of metal, nylon, Kevlar, carbon, technopolymer ;
- a set of coned-disc springs joined to each other with their edges, arranged in an alternate way in succession, therefore the large base of the first coned-disc spring is joined to the large base of a subsequent second coned-disc spring, and the tight base of this latter is joined to the tight base of a subsequent third coned-disc spring, and so the remaining coned-disc springs that compose the entire lengthened elastic element 14.

Such an elastic element 14 may also be constitute by any other type of lengthened elastic and internally hollow body.

The steering means 13, lengthened upward, is constituted by a central lengthened body 56, shaped with a structure almost box-shaped provided with an external seat along one vertical front or back wall thereof, adapted to house in a removable manner one means for supplying power energy 57, as for example a rechargeable battery, adapted to power all the electrical and electronic components of the device 10, such a central lengthened body 56 being connected with its lower end portion 58 to the upper end portion 55 f the jointing element 14 and being provided at its upper end portion 59 with a shaped handle 60 and one control and settings panel 61 for the device 10.

The power supply, in addition to the means for supplying power energy 57, may be provided by the electrical domestic grid by means of electrical cables and on electrical plug (not shown) arranged on the lengthened body 56.

Such a handle 60 is of the closed ring type and is extended upward with two first lengthened segments 62 identical and specular to each other and inclined slightly outward, and continue with two second lengthened segments 63 identical e specular to each other and extended frontally, and close itself with a horizontal lengthened segment 64 which connect the upper en portion of two second segments 63.

This conformation of the handle 60 allows to the operator to grab it with one or two hands in the point that is suitable for him depending on the inclination of the steering means 13, that is this latter is basically in a vertical position thereof and the operator grabs the first two lengthened segments 62, when the steering means will be in a inclined position thereof, hence the handle 60 will be close to the walkable surface 11, the operator, for not bowing and tiring too much, will grab the third horizontal segment 64, which will be in a raised position with respect to the other segments.

The control and settings panel 61 is adapted to activate the device 10 and to select a operational program loaded in advance for the electrical and electronic component elements and means above described, and is constituted by at least a display, where the data of the device 10 are visible, and a plurality of setting means, as for example push-buttons and/or knobs and/or touch screen commands, which are built-in in the above display, furthermore, such a panel 61 is associated with at least a first electronic control and command means (not shown), constituted as for example by an electronic circuit board equipped with a microprocessor, housed inside the lengthened body 56 and powered by the battery 57.

Said first control and command means us adapted to set the activation and the power-down of the device 10 and to set the operational program loaded in advance of the electrical and electronic means comprised in the device, as for example the intensity of the operation, the direction of the rotation of the brushing means 21 and 22, etc..

Said first control and command means may be also associated to at least a second electronic control and command means (not shown), constituted as for example by an electronic circuit board equipped with at least a microprocessor too, and positioned on the frame 16, which second control and command means is adapted to receive instructions from the first control and command means and to adjust the activation and the shot-down of the electric and electronic means comprised in the device, to carry out the operational program and to adjust the intensity of operation.

Said first control and command means is also associated to the sensor means 53, for being able to carry out the above the described operations, particularly, said first control and command means receives the data from the sensor means 53 and sends command to the second control and command means.

Said first control and command means is also associated to at least an additional detecting means (not shown) positioned in the upper end portion 59 of the steering means and adapted to detect the presence of the operator in close proximity to the device 10, in such a way that if the device 10 is in the operational state and the operator is not detected, said first control and command means sends the command to the second electronic control and command means to deactivate all the electrical and electronic means, which are operative in that moment, for then re-activate them when the operator is detected.

Such an additional detecting means is constitute by at least a proximity sensor, that may be of the ultrasound type or of the optical type and position in the area of the control and setting panel 61, thereby be able to detect the presence of the operator within a determined distance, and/or may be of the capacitive type and position on the handle 60, thereby be able to detect if the operator is grabbing or not said handle 60 with at least one hand.

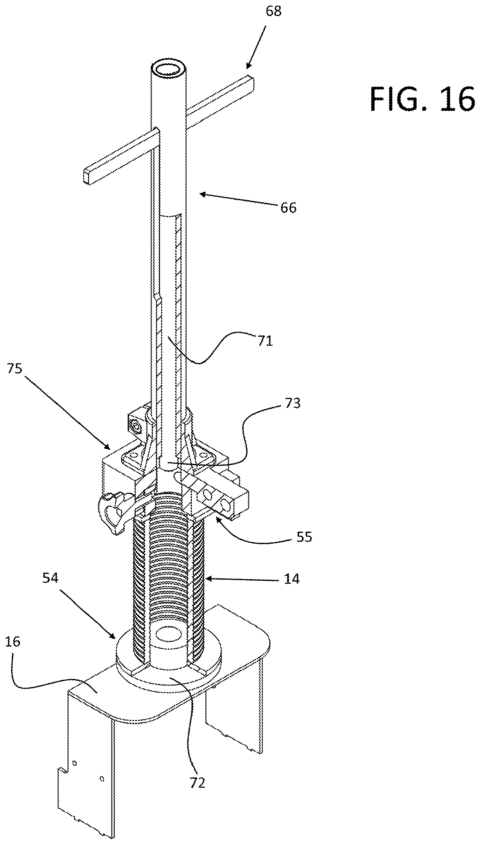

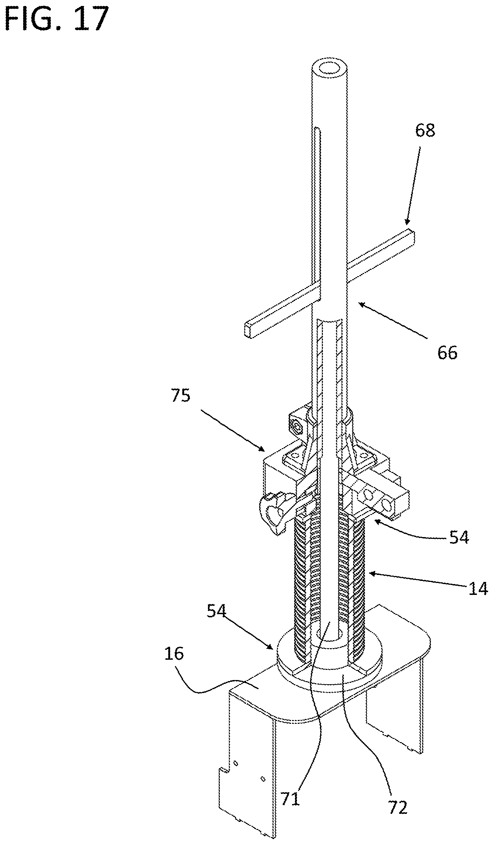

Said box-shaped structure of the lengthened body 56 is provided inside with a lengthened fixing means 66 (See Figs. 16-17), constituted by a vertical rod 71 to which an horizontal rod 68 is fixed on its the upper part and housed in a sliding manner within a vertical hollow guide member 67, and moved by the operator by means of the horizontal rod 68, which is extended with at least one its side end portion beyond the side vertical walls of the body 56, by means of two vertical through slots 70 provided on to this latter and specular to each other.

In the starting position of the device 10, such a fixing means 66 from a rest raised position thereof, in which it is maintained by means of fixing means of the magnetic type or by means of a pawl wherein it is engaged the horizontal rod 68, may slide downward by the operator in an operational lowered position thereof passing through the opened end portion 58 and stopping at the internal base of the jointing means 14, where an end-of-stroke element 72 is positioned, constituted preferably by a vertical hollow cylinder into which the lower end portion 73 of the same fixing means 66 engage itself, in this manner the device 10 is locked in the starting position thereof, for be able to be stored, therefor by avoiding the possibility that the jointing means 14 may be curved.

A plurality of wheels 74 may be fixed under the frame 16 in a raised manner, preferably in front with respect to the brushing means 21 and 22, in such a way that when the fixing means 66 is in the operational position thereof, the operator may incline the device 10 by leant such wheels 74 on the walkable surface 11, therefore it is possible to easily transport it without that the brushes 21 and 22 and the restraining means 29 create friction, since they are in a raised position thereof.

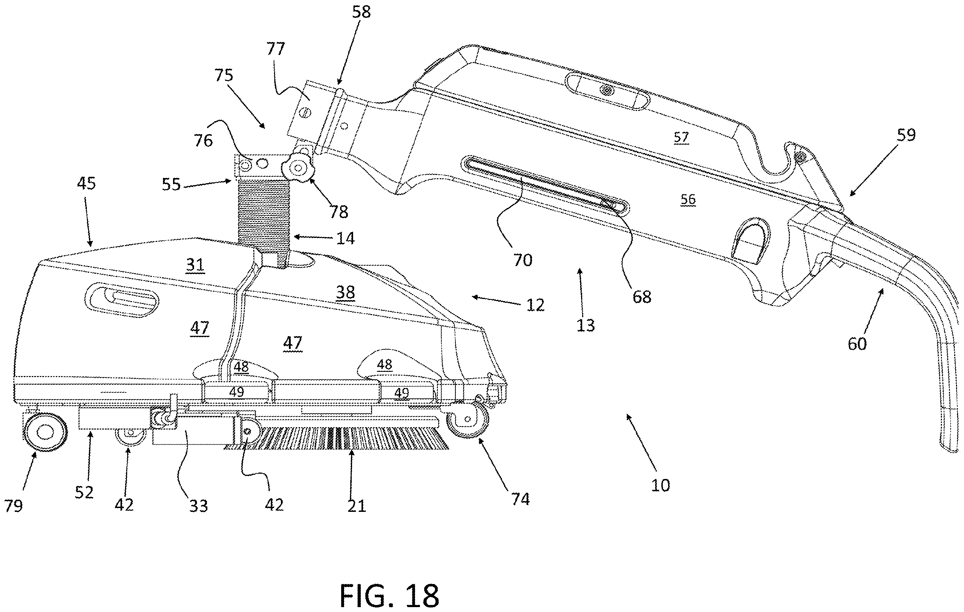

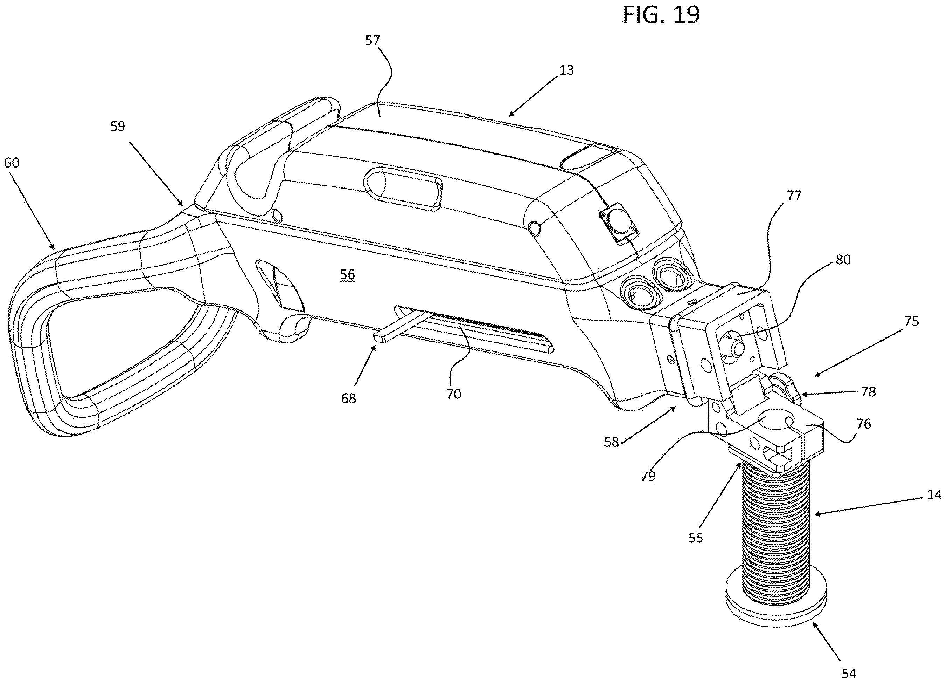

Said lengthened body 56 may be connected with its lower end portion 58 to the jointing element 14 by means of a joining means 75, which is lockable in position and adapted to maintain the steering means 13 in an operational position thereof or to release it from such a position for allowing it to be inclined without to intervene on the jointing element 14 (See Figs. 18, 19), therefore for being able to reduce the overall dimensions and packaging or storing it easily.

Such a joining means 75 is constituted by a lower part 76 connected by means of a horizontal pivot to an upper part 77, in such a way that two parts are tilting to each other, and may be locked in position, when such two parts 76 and 77 are adjacent one above the other, by means of a locking means 78, constituted as for example by a bolt.

The lower part 76 is fixed to the upper end portion of the jointing element 14, whereas the upper part 77 is fixed to the lower end portion 58 of the lengthened body 56 of the steering means 13. Said parts 76 and 77 are each provided with a respective vertical concentric through hole 79, 80 (See Fig. 19), adapted to allow the passage of the vertical rod 71 of the fixing means 66.

In addition to the aims and advantageous above listed, such a device has the advantageous characteristic that by using a jointing means 14, that is of the elastic type, the vibrations created by the device 10 during its operational state, or possible impacts with external objects, are absorbed by the same jointing means 14 that twist automatically for return then in the position thereof in which it was before the impact, without so weigh on the operator, therefore who may work with small loss of energy and with few traumas.

## Claims

1. Device (10) realized as scrubber-dryer machine for cleaning walkable surfaces (11), as for example floors, pavements, asphalted roads, etc. for cleaning walkable surfaces, adapted to move forward automatically and to be steered manually by an operator, and constituted by a cleaning lower base body (12) connected on its upper part by means of a movable jointing element (14) to a steering means lengthened upward (13), said lower base body (12) comprising a frame (16) to which one or more lower brushing means (21, 22) are fixed to and actuated in rotation by means of respective electrical actuating means (27, 28), and adapted to brush the walkable surfaces (11), by removing the dirty from it and to create a friction with the walkable surface (11) such to move forward automatically in a rectilinear way the device (10), at least a wetting system fixed to the frame (16), and positioned in correspondence of said brushing means (21, 22) and adapted to wet them, and at least a dirty collection system fixed to the frame (16), positioned on the back of said brushing means (21, 22) with respect the travel direction of the device (10) and adapted to collecting the dirty brushed by the same brushing means, said wetting system being constituted by one containment means (38), adapted to contain the cleaning liquid, one electrical pumping means (39), adapted to draw the cleaning liquid, and delivery means (40) for delivering such a liquid, said dirty collection system being constituted by at least one restraining means (29), adapted to restrain the dirty conveyed by the brushing means (21, 22), at least one suction means (30), adapted to suck the restrained dirty, and at least a collection means (31), adapted to contain the sucked dirty, said steering means (13) comprising an upward lengthened body (56) joined at the top thereof to a handle (60), and being of the inclinable type in any direction from a starting position perpendicular with respect to the lower body (12) and being adapted to steer the device (10), **characterized in that** said jointing element (14) is constituted by a lengthened elastic and internally hollow push element (14), constrained with its lower end portion (54) to the frame (16), in the barycenter point of the lower body (12) and with its upper end portion (55) to the steering means (13), and that from the rest position thereof, in which it has a rectilinear upward extension, it may be curved downward by applying a downward and outward push force to the upper end portion (55) thereof and in any direction with respect to its vertical axis when it is in the rest position thereof, therefore to incline downward the steering means (13), and in this curved position being able to rotate along the vertical axis of the rest position of the same elastic element, without that the lower body (12) be interested by such a rotation, by decreasing the downward push force of the upper end portion (55), the elastic element (14) returning automatically toward the rest position thereof, such an elastic element (14) being also adapted to receive a twisting moment to its upper end portion (55), by means of said steering means (13), for then applying said twisting moment, with its lower end portion (54), to the frame (16) of the lower body (12), therefore this latter rotates on its perpendicular axis extent upward of the angle desired by the operator, without be inclined.

2. Device (10) according to claim 1, **characterized in that** such a lengthened elastic and internally hollow push element (14) is constituted by a sprung element, as for example at least one spring, made of metal or polymer.

3. Device (10) according to claim 2, **characterized in that** said sprung element may be constituted by at least two springs inserted or co-moulded concentrically one inside the other, made of metal and/or polymer.

4. Device (10) according to claim 2, **characterized in that** said sprung element may be constituted by one metal bellow, as for example made of steel, with a core made of elastic and semi-rigid material, as for example rubber.

5. Device (10) according to claim 2, **characterized in that** said sprung element may be constituted by a pipe made of elastic and semi-rigid material, as for example rubber, provided with an internal reinforcement made of reinforcing fibers woven to each other, as for example wire made of metal, nylon, Kevlar, carbon, technopolymer.

6. Device (10) according to claim 2, **characterized in that** said sprung element may be constituted by a set of coned-disc springs joined to each other with their edges, arranged in an alternate way in succession, therefore the large base of the first coned-disc spring is joined to the large base of a subsequent second coned-disc spring, and the tight base of this latter is joined to the tight base of a subsequent third coned-disc spring, and so the remaining coned-disc springs that compose the entire lengthened elastic element (14).

7. Device (10) according to claim 1, **characterized in that** said electrical actuating means (27, 28), constituted as for example by electrical motors, are arranged on the upper part of said frame (16), and above to the respective brushing means (21 and 22), that they have to actuate, said brushing means (21, 22) being arranged side by side to each other in a transversal manner with respect the orientation of the lower body (12), and rotating in opposite directions to each other along an axis which is slightly inclined inward with respect to the vertical of the device (10) leant on a horizontal plane.

8. Device (10) according to claim 1, **characterized in that** said steering means (13) comprises at the top thereof one control and settings panel (61) associated with at least a first electronic control and command means for activating the device (10) and to select an operational program, loaded in advance in the first electronic control and command means, of the electrical and electronic component elements of the same device (10), said control and setting panel (61) being constituted by at least a display, where the data of the device (10) are visible, and a plurality of setting means, as for example push-buttons and/or knobs and/or touch screen commands, which are built-in in the above display.

9. Device (10) according to claim 8, **characterized in that** said first control and command means is associated to at least a second electronic control and command means, constituted as for example by an electronic circuit board equipped with at least a microprocessor too, and positioned on the frame (16), which second control and command means being adapted to receive instructions from the first control and command means and to adjust the activation and the shot-down of the electric and electronic means comprised in the device (10) and to carry out the selected operational program and to adjust the intensity of operation.

10. Device (10) according to claim 1, **characterized in that** said a handle (60) is of the closed ring type and is extended upward with two first lengthened segments (62) identical and specular to each other and inclined slightly outward, and continue with two second lengthened segments (63) identical e specular to each other and extended frontally, and close itself with a horizontal lengthened segment (64) which connect the upper en portion of two second segments (63).

11. Device (10) according to claim 1, **characterized in that** said restraining means (29) for the dirty is constituted by a lengthened semi-circular support (29') provided with at least a vertical through hole (32) and which supports on its lower edge at least a lengthened element (33), made of a semi-rigid material, as for example rubber, said support (29') being positioned transversally with respect to the orientation of the device (10), with its concave part (34) turned toward the two brushing means (21 and 22), and being connected to the lower part of the frame (16) by means of at least two pair of levers (35, 36), pivoted at their end portions (37) parallel to each other, and arranged longitudinally with respect to the orientation of the device (10), which levers (35, 36) being adapted to rotate on their end portions (37) according the vertical movement caused by the restraining means (29) that follows the conformation of the walkable surface (11), or by being lifted or lowered manually or automatically when the operator considers it necessary, said restraining means (29) being kept lifted by means of quick fixing means or magnetic means or electro-magnetic means, or equivalent.

12. Device (10) according to claim 11, **characterized in that** said levers (35, 36) are made of elastic metal, preferably of harmonic steel, and are structured and adapted to be bent transversally when the restraining means (29) bumps laterally against an external obstacle (80), for then returning automatically in the original position thereof after having passed such an external obstacle.

13. Device (10) according to claim 11, **characterized in that** said levers (35, 36) may be rotated vertically by means of one electrical actuator means (51), placed on the upper part of the frame (16).

14. Device (10) according to claim 1, **characterized in that** said restraining means (29) are provided with two support wheels (42), placed laterally and/or centrally with respect to the lengthened support (29') and are fixed by means of metallic brackets (43).

15. Device (10) according to claim 11, **characterized in that** said suction means (30) is of the electrical type, and is arranged on the upper part of said frame (16) and above said restraining means (29), and with its inlet mouth is connected in a communicating way to said vertical through hole (32), and with its outlet mouth is connected in a communicating manner to said collection means (31).

16. Device (10) according to claim 15, **characterized in that** said collection means (31) is constituted by a first tank arranged in a removable manner above the back half portion of said frame (16) and the components placed in this portion of frame, and has a base that follows the outline and the dimensions of the portion of frame on which is leant and a height that doesn't interfere with the jointing element (14).

17. Device (10) according to claim 1, **characterized in that** said electrical pumping means (39), constituted for example by one electrical pump, is placed on the frame (16), in the central area of this latter, and is associated in a communicating manner with said containment means (38), which is constituted nu a second tank arranged in a removable manner above the front half portion of said frame (16) and the components placed in this portion of frame, and has a base that follows the outline and dimensions of the portion of frame on which it is leant and an height that doesn't interfere with the jointing element (14).

18. Device (10) according to claims 15 and 17, **characterized in that** the first tank (31) and the second tank (38) are joined to each other and form a unique body (45) having an upper surface (46) almost continuous and inclined frontally downward and side walls (47) inclined inward and upward, and finally having a vertical hole (41), in correspondence of the jointing element (14), in such a way that this latter be inserted therein and be free to carry out its function.

19. Device (10) according to claim 18, **characterized in that** said body (45), along its lower base outline, is also provided with a plurality of shape recesses (48) identical and equidistant to each other, in correspondence of each of which, above said frame (16), a respective impact resistant means (49) is housed and protruded partially outward the device (10), such an impact resistant means (49) is constituted for example by a roller pivoted with a vertical rotation axis, free in rotation, and made of semi-rigid elastic material, as for example rubber.

20. Device (10) according to one of preceding claims, **characterized in that** at least one electrical sanitation means (52) is fixed to the lower part of the frame (16), at the back of said restraining means (29) with respect to the direction of advancement of said device (10), such a sanitation means (52) being constituted as for example by one or more UV lamps and being adapted to complete the sanitation of the walkable surface (11) just treated, and at least a pivoting wheel (79) being positioned in the lower part of the frame (16), at the back with respect to said electrical sanitation means (52), and being adapted to support the back part of the lower body (12), by avoiding that this latter is inclined backward.

21. Device (10) according to claim 8, **characterized in that** said first electronic control and command means is also associated to at least an additional first detecting means, positioned on the upper part of the steering means (13) and adapted to detect the presence of the operator in close proximity to the device (10), in such a way that if the device (10) is in the operational state thereof and the operator is not detected, said first electronic control and command means sends the command to the second electronic control and command means to deactivate all the electrical and electronic means, which are operative **in that** moment, for then re-activating them when the operator will be detected.

22. Device (10) according to claim 21, **characterized in that** said first detecting means is constitute by a proximity sensor of the ultrasound type or of the optical type and is position in the area of the control and setting panel (61).

23. Device (10) according to claim 21, **characterized in that** said first detecting means is constitute by a proximity sensor of the capacitive type and position on the handle (60).

24. Device (10) according to one of preceding claims, **characterized in that** said first control and command means is also associated to the sensor means (53), constituted for example by a sensor of the gyroscope type or inertial type, positioned on the frame (16), and adapted to detect the direction of advancement of the device (10), that is if the operator is pushing or pulling the same device (10) for inverting the direction of advancement thereof, therefore the brushing means (21 and 22) may eventually invert the own direction of rotation and that the possible restraining means (29) positioned on the front with respect to the brushing means (21 and 22) with respect to the advancement direction of the device (10) be lifted up from the walkable surface (11).

25. Device (10) according to claim 1, **characterized in that** said box-shaped structure of the lengthened body (56) is provided inside with a lengthened fixing means (66), which may slide vertically from a raised rest position to an lowered operational position thereof, and vice versa, for locking or unlocking the device (10) in the starting position thereof.

26. Device (10) according to claim 25, **characterized in that** said lengthened fixing means (66) is constituted by a vertical rod (71) to which an horizontal rod (68) is fixed on its the upper part and housed in a sliding manner within a vertical hollow guide member (67), and moved by the operator by means of the horizontal rod (68), which is extended with at least one its side end portion beyond the side vertical walls of the body (56), by means of two vertical through slots (70) provided on to this latter and specular to each other, said lengthened fixing means (66) being maintained in the raised rest position thereof by means of fixing means of the magnetic type or by means of a pawl wherein it is engaged the horizontal rod (68), said lengthened fixing means (66), in the operational lowered position thereof, is engaged with its lower end portion (73) into an end-of-stroke element (72), positioned inside the base of the jointing element (14).

27. Device (10) according to claim 26, **characterized in that** a plurality of wheels (74) is fixed under the frame (16) in a raised manner, preferably in front with respect to the brushing means (21 and 22), in such a way that when the fixing means (66) is in the operational position thereof, the operator may incline the device (10) by leant such wheels (74) on the walkable surface (11), therefore it is possible to easily transport it with the brushes (21 and 22) and the restraining means (29) that are in a raised position thereof.

28. Device (10) according to one of preceding claims, **characterized in that** said lengthened body (56) is connected with its lower end portion (58) to the upper end portion of the jointing element (14) by means of a joining means (75), which is lockable in position and adapted to maintain the steering means (13) in an operational position thereof or to release it from such a position for allowing it to be inclined without to intervene on the jointing element (14), in a way to reduce the overall dimensions and packaging or storing it, such a joining means (75) being constituted by a lower part (76) connected in a tilting manner to an upper part (77), that and may be locked in adjacent position, one above the other, by means of a locking means (78), constituted as for example by a bolt.

29. Device (10) according to claim 28, **characterized in that** said parts (76 and 77) are each provided with a respective vertical concentric through hole (79, 80), adapted to allow the passage of the vertical rod (71) of the fixing means (66).

30. Device (10) according to one of preceding claims, **characterized in that** said lengthened body (56) is provided with an external seat along one vertical front or back wall thereof, adapted to house in a removable manner one means for supplying power energy (57), as for example a rechargeable battery, adapted to power all the electrical and electronic components of the device (10).

## Patentansprüche

1. Vorrichtung (10), als Scheuersaugmaschine hergestellt, zum Reinigen von begehbaren Flächen (11), wie beispielweise Fußböden, Gehwegen, asphaltierten Straßen, usw. zum Reinigen von begehbaren Oberflächen, die eingerichtet ist, sich automatisch vorwärts zu bewegen und von einem Bediener manuell gesteuert zu werden, und die aus einem unteren Reinigungsgrundkörper (12) besteht, der an seinem oberen Teil mittels eines beweglichen Verbindungselements (14) mit einem nach oben verlängerten Steuermittel (13) verbunden ist, wobei der untere Grundkörper (12) einen Rahmen (16) umfasst, an dem ein oder mehrere untere Bürstenmittel (21, 22) befestigt sind und mittels entsprechender elektrischer Betätigungsmittel (27, 28) in Drehung versetzt werden, und die eingerichtet sind, die begehbaren Oberflächen (11) zu bürsten, indem sie den Schmutz davon entfernen und eine Reibung mit der begehbaren Oberfläche (11) erzeugen, so dass die Vorrichtung (10) automatisch geradlinig vorwärts bewegt wird, mindestens ein Befeuchtungssystem, das an dem Rahmen (16) befestigt ist und an den Bürstenmitteln (21, 22) positioniert ist und das eingerichtet ist, diese zu befeuchten, und mindestens ein Schmutz-Sammelsystem, das an dem Rahmen (16) befestigt ist und auf der Rückseite der Bürstenmittel (21, 22) in Bezug auf die Bewegungsrichtung der Vorrichtung (10) positioniert ist und das eingerichtet ist, den durch dieselben Bürstenmittel gebürsteten Schmutz zu sammeln, wobei das Befeuchtungssystem aus einem Aufnahmemittel (38), das eingerichtet ist, die Reinigungsflüssigkeit aufzunehmen, einem elektrischen Pumpenmittel (39), das eingerichtet ist, die Reinigungsflüssigkeit zu entnehmen, und Versorgungsmittel (40) zur Versorgung einer solchen Flüssigkeit besteht, wobei das Schmutz-Sammelsystem aus mindestens einem Rückhaltemittel (29), das eingerichtet ist, den von den Bürstenmitteln (21, 22) beförderten Schmutz zurückzuhalten, mindestens einem Saugmittel (30), das eingerichtet ist, den zurückgehaltenen Schmutz anzusaugen, und mindestens einem Sammelmittel (31), das eingerichtet ist, den angesaugten Schmutz aufzunehmen besteht, wobei das Steuermittel (13) einen nach oben verlängerten Körper (56) umfasst, der an seinem oberen Ende mit einem Griff (60) verbunden ist und von der Art ist, dass er aus einer Ausgangsposition, die senkrecht in Bezug auf den unteren Körper (12) ist, in jede Richtung geneigt werden kann und das eingerichtet ist, die Vorrichtung (10) zu steuern, **dadurch gekennzeichnet, dass** das Verbindungselement (14) aus einem verlängerten elastischen und innen Hohlschubelement (14) besteht, das mit seinem unteren Endabschnitt (54) am Rahmen (16) im Schwerpunkt des unteren Körpers (12) und mit seinem oberen Endabschnitt (55) an dem Steuermittel (13) befestigt ist, und dass es aus seiner Ruheposition, in der er eine geradlinige Ausdehnung nach oben aufweist, nach unten gekrümmt werden kann, indem eine nach unten und außen gerichtete Schubkraft auf seinen oberen Endabschnitt (55) ausgeübt wird, und zwar in jeder Richtung in Bezug auf seine vertikale Achse, wenn er sich in seiner Ruheposition befindet, um deshalb das Steuermittel (13) nach unten zu neigen, und in dieser gekrümmten Position in der Lage ist, sich entlang der vertikalen Achse der Ruheposition desselben elastischen Elements zu drehen, ohne dass der untere Körper (12) durch eine solche Drehung beeinflusst wird, indem die nach unten gerichtete Schubkraft des oberen Endabschnitts (55) verringert wird, wobei das elastische Element (14) automatisch in seine Ruheposition zurückkehrt, wobei ein solches elastisches Element (14) auch eingerichtet ist, ein Drehmoment auf seinen oberen Endabschnitt (55) mittels des Steuermittels (13) aufzunehmen, um dann das Drehmoment mit seinem unteren Endabschnitt (54) auf den Rahmen (16) des unteren Körpers (12) auszuüben, so dass dieser letztere sich um seine senkrechte Achse um den vom Bediener gewünschten Winkel nach oben dreht, ohne geneigt zu sein.

2. Vorrichtung (10) nach Anspruch 1, **dadurch gekennzeichnet, dass** ein solches verlängertes elastisches und innen Hohlschubelement (14) aus einem Federelement, wie beispielweise mindestens einer Feder, aus Metall oder Polymer besteht.

3. Vorrichtung (10) nach Anspruch 2, **dadurch gekennzeichnet, dass** das Federelement aus mindestens zwei konzentrisch ineinander eingefügten oder mitgeformten Federn aus Metall und/oder Polymer bestehen kann.

4. Vorrichtung (10) nach Anspruch 2, **dadurch gekennzeichnet, dass** das Federelement aus einem Metallbalg, wie beispielweise aus Stahl, mit einem Kern aus elastischem und halbsteifem Material, beispielweise Gummi, bestehen kann.

5. Vorrichtung (10) nach Anspruch 2, **dadurch gekennzeichnet, dass** das Federelement aus einem Rohr aus elastischem und halbsteifem Material, wie beispielweise Gummi, bestehen kann, das mit einer inneren Verstärkung bestehend aus miteinander gewobenen Verstärkungsfasern, wie beispielweise Draht aus Metall, Nylon, Kevlar, Kohlenstoff, Technopolymer, versehen ist.

6. Vorrichtung (10) nach Anspruch 2, **dadurch gekennzeichnet, dass** das Federelement aus einem Satz von kegelscheibenförmigen Federn bestehen kann, die mit ihren Rändern miteinander verbunden sind und abwechselnd hintereinander angeordnet sind, sodass der große Boden der ersten kegelscheibenförmigen Feder mit dem großen Boden einer nachfolgenden zweiten kegelscheibenförmigen Feder verbunden ist, und der enge Boden dieser letzteren mit dem engen Boden einer nachfolgenden dritten kegelscheibenförmigen Feder verbunden ist, und so die übrigen kegelscheibenförmigen Feder das gesamte verlängerte elastische Element (14) bilden.

7. Vorrichtung (10) nach Anspruch 1, **dadurch gekennzeichnet, dass** die elektrischen Betätigungsmittel (27, 28), die beispielsweise aus Elektromotoren bestehen, am oberen Teil des Rahmens (16) und oberhalb der jeweiligen Bürstenmittel (21 und 22), die sie betätigen müssen, angeordnet sind, wobei die Bürstenmittel (21, 22) quer zur Ausrichtung des unteren Körpers (12) nebeneinander angeordnet sind und sich in entgegengesetzten Richtungen zueinander entlang einer Achse drehen, die in Bezug auf die Vertikale der an einer horizontalen Ebene anliegenden Vorrichtung (10) leicht nach innen geneigt ist.

8. Vorrichtung (10) nach Anspruch 1, **dadurch gekennzeichnet, dass** das Steuermittel (13) an seiner Oberseite eine Steuer-und Einstellungstafel (61) umfasst, die mindestens einem ersten elektronischen Steuer-und Bedienungsmittel zugeordnet ist, um die Vorrichtung (10) zu betätigen und ein Betriebsprogramm auszuwählen, das im Voraus in das erste Steuer-und Bedienungsmittel der elektrischen und elektronischen Bestandteilenelemente derselben Vorrichtung (10) geladen wurde, wobei die Steuer-und Einstellungstafel (61) aus mindestens einer Anzeige, auf der die Daten der Vorrichtung (10) sichtbar sind, und einer Mehrzahl von Einstellmitteln, wie beispielweise Drucktasten und/oder Knöpfen und/oder Touchscreen-Befehlen, die in der obigen Anzeige eingebaut sind, besteht.

9. Vorrichtung (10) nach Anspruch 8, **dadurch gekennzeichnet, dass** das erste Steuer-und Bedienungsmittel mindestens einem zweiten elektronischen Steuer-und Bedienungsmittel zugeordnet ist, das beispielsweise aus einer elektronischen Leiterplatte besteht, die ebenfalls mit mindestens einem Mikroprozessor ausgestattet ist, und das auf dem Rahmen (16) angeordnet ist, wobei das zweite Steuer-und Bedienungsmittel so eingerichtet ist, dass es Befehle von dem ersten Steuer-und Bedienungsmittel empfängt und die Aktivierung und die Abschaltung der in der Vorrichtung (10) enthaltenen elektrischen und elektronischen Mittel reguliert und das gewählte Betriebsprogramm ausführt und das Betriebsniveau reguliert.

10. Vorrichtung (10) nach Anspruch 1, **dadurch gekennzeichnet, dass** ein Griff (60) als geschlossener Ring ausgebildet ist und sich nach oben mit zwei ersten verlängerten Segmenten (62) erstreckt, die identisch und spiegelbildlich zueinander sind und leicht nach außen geneigt sind, und sich mit zwei zweiten verlängerten Segmenten (63) fortsetzt, die identisch und spiegelbildlich zueinander sind und sich nach vorne erstrecken, und sich mit einem horizontalen verlängerten Segment (64) schließt, das den oberen Endabschnitt der beiden zweiten Segmente (63) verbindet.

11. Vorrichtung (10) nach Anspruch 1, **dadurch gekennzeichnet, dass** das Rückhaltemittel (29) für den Schmutz aus einem verlängerten halbkreisförmigen Träger (29') besteht, der mit mindestens einer vertikalen Durchgangsbohrung (32) versehen ist und der an seinem unteren Rand mindestens ein verlängertes Element (33) aus einem halbstarren Material wie beispielweise Gummi trägt, wobei der Träger (29') quer zur Ausrichtung der Vorrichtung (10) positioniert ist, wobei sein konkaver Teil (34) den beiden Bürstenmitteln (21 und 22) zugewandt ist, und wobei er mit dem unteren Teil des Rahmens (16) mittels mindestens zwei Hebelpaaren (35, 36) verbunden ist, wobei die Hebel an ihren Endabschnitten (37) parallel zueinander schwenkbar sind und in Längsrichtung in Bezug auf die Ausrichtung der Vorrichtung (10) angeordnet sind, wobei die Hebel (35, 36) eingerichtet sind, sich an ihren Endabschnitten (37) entsprechend der vertikalen Bewegung, die durch das Rückhaltemittel (29) verursacht wird, das der Struktur der begehbaren Oberfläche (11) folgt, zu drehen oder indem sie manuell oder automatisch angehoben oder abgesenkt werden, wenn der Bediener es für notwendig hält, wobei das Rückhaltemittel (29) mittels Schnellbefestigungsmitteln oder magnetischen Mitteln oder elektromagnetischen Mitteln oder ähnlich angehoben gehalten wird.

12. Vorrichtung (10) nach Anspruch 11, **dadurch gekennzeichnet, dass** die Hebel (35, 36) aus elastischem Metall, vorzugsweise aus harmonischem Stahl, bestehen und so strukturiert und eingerichtet sind, dass sie quer gebogen werden, wenn das Rückhaltemittel (29) seitlich gegen ein äußeres Hindernis (80) stößt, um dann automatisch in die ursprüngliche Position zurückzukehren, nachdem sie ein solches äußeres Hindernis überwunden haben.

13. Vorrichtung (10) nach Anspruch 11, **dadurch gekennzeichnet, dass** die Hebel (35, 36) mittels eines elektrischen Betätigungsmittels (51), die auf dem oberen Teil des Rahmens (16) angeordnet ist, vertikal gedreht werden können.

14. Vorrichtung (10) nach Anspruch 1, **dadurch gekennzeichnet, dass** die Rückhaltemittel (29) mit zwei Stützrädern (42) versehen sind, die seitlich und/oder mittig in Bezug auf den verlängerten Träger (29') angeordnet sind und mittels metallischer Bügel (43) befestigt sind.

15. Vorrichtung (10) nach Anspruch 11, **dadurch gekennzeichnet, dass** das Ansaugmittel (30) elektrisch ist und am oberen Teil des Rahmens (16) und oberhalb des Rückhaltemittels (29) angeordnet ist und mit seiner Einlassöffnung in kommunizierender Weise mit der vertikalen Durchgangsbohrung (32) verbunden ist und mit seiner Auslassöffnung in kommunizierender Weise mit dem Sammelmittel (31) verbunden ist.

16. Vorrichtung (10) nach Anspruch 15, **dadurch gekennzeichnet, dass** das Sammelmittel (31) aus einem ersten Behälter besteht, der abnehmbar über dem hinteren Halbabschnitt des Rahmens (16) und den in diesem Rahmenabschnitt positionierten Bauteilen angeordnet ist und einen Boden aufweist, der dem Umriss und den Abmessungen des Rahmenabschnitts folgt, an dem er anliegt, und eine Höhe aufweist, die nicht das Verbindungselement (14) stört.

17. Vorrichtung (10) nach Anspruch 1, **dadurch gekennzeichnet, dass** das elektrische Pumpenmittel (39), das beispielsweise aus einer elektrischen Pumpe besteht, auf dem Rahmen (16) im mittleren Bereich dieses letzteren angeordnet ist und dem Rückhaltemittel (38) in kommunizierender Weise zugeordnet ist, die aus einem zweiten Behälter besteht, der abnehmbar über dem vorderen Halbabschnitt des Rahmens (16) und den in diesem Rahmenabschnitt positionierten Bauteilen angeordnet ist, und einen Boden aufweist, der dem Umriss und den Abmessungen des Rahmenabschnitts folgt, an dem er anliegt, und eine Höhe aufweist, die das Verbindungselement (14) nicht stört.

18. Vorrichtung (10) nach den Ansprüchen 15 und 17, **dadurch gekennzeichnet, dass** der erste Behälter (31) und der zweite Behälter (38) miteinander verbunden sind und einen einzigen Körper (45) bilden, der eine obere Fläche (46) aufweist, die fast durchgehend und vorne nach unten geneigt ist, und Seitenwände (47), die nach innen und nach oben geneigt sind, und schließlich eine vertikale Bohrung (41) aufweist, die sich an dem Verbindungselement (14) befindet, so dass dieses letztere darin eingesetzt werden kann und frei ist, seine Funktion durchzuführen.

19. Vorrichtung (10) nach Anspruch 18, **dadurch gekennzeichnet, dass** der Körper (45) entlang seines unteren Grundrisses auch mit einer Mehrzahl von Form-Vertiefungen (48) versehen ist, die identisch und sich in gleichem Abstand zueinander befinden, an jeder von ihnen über dem Rahmen (16) ein entsprechendes stoßfestes Mittel (49) aufgenommen ist und teilweise aus der Vorrichtung (10) vorsteht, wobei ein solches stoßfestes Mittel (49) beispielweise aus einer Rolle besteht, die mit einer vertikalen Drehachse schwenkbar ist, frei drehbar ist und aus einem halbstarren elastischen Material, wie beispielweise Gummi, besteht.

20. Vorrichtung (10) nach einem der vorhergehenden Ansprüche, **dadurch gekennzeichnet, dass** mindestens ein elektrisches Sanierungsmittel (52) an dem unteren Teil des Rahmens (16) hinter dem Rückhaltemittel (29) in Bezug auf die Vorschubrichtung der Vorrichtung (10) befestigt ist, wobei ein solches Sanierungsmittel (52) beispielsweise aus einer oder mehreren UV-Lampen besteht und eingerichtet ist, die Sanierung der gerade behandelten begehbaren Oberfläche (11) zu vervollständigen, und mindestens ein schwenkbares Rad *(79)* , das im unteren Teil des Rahmens (16) hinten in Bezug auf dem elektrischen Sanierungsmittel (52) positioniert ist und eingerichtet ist, den hinteren Teil des unteren Körpers (12) zu stützen, sodass es verhindert wird, dass dieser letztere nach hinten geneigt ist.

21. Vorrichtung (10) nach Anspruch 8, **dadurch gekennzeichnet, dass** das erste elektronische Steuer-und Bedienungsmittel auch mindestens einem zusätzlichen ersten Erfassungsmittel zugeordnet ist, die auf dem oberen Teil des Steuermittels (13) angeordnet ist und eingerichtet ist, die Anwesenheit des Bedieners in unmittelbarer Nähe der Vorrichtung (10) zu erfassen, derart, dass, wenn sich die Vorrichtung (10) in ihrem Betriebszustand befindet und den Bediener nicht erfasst wird, das erste elektronische Steuer-und Bedienungsmittel den Befehl an das zweite elektronische Steuer-und Bedienungsmittel sendet, um alle elektrischen und elektronischen Mittel, die in diesem Moment in Betrieb sind, zu deaktivieren, um sie dann wieder zu aktivieren, wenn der Bediener erfasst wird.

22. Vorrichtung (10) nach Anspruch 21, **dadurch gekennzeichnet, dass** das erste Erfassungsmittel aus einem Näherungssensor des Ultraschalltyps oder des optischen Typs besteht und im Bereich der Steuer-und Einstelltafel (61) angeordnet ist.

23. Vorrichtung (10) nach Anspruch 21, **dadurch gekennzeichnet, dass** das erste Erfassungsmittel aus einem Näherungssensor des kapazitiven Typs besteht und auf dem Griff (60) positioniert wird.

24. Vorrichtung (10) nach einem der vorhergehenden Ansprüche, **dadurch gekennzeichnet, dass** das erste Steuer-und Bedienungsmittel auch dem Sensormittel (53) zugeordnet ist, das beispielweise aus einem Sensor vom Typ Gyroskop oder aus einem Trägheitssensor besteht, der auf dem Rahmen (16) positioniert ist und eingerichtet ist, die Vorschubrichtung der Vorrichtung (10) zu erfassen, das heißt, wenn der Bediener dieselbe Vorrichtung (10) schiebt oder zieht, um deren Vorschubrichtung umzukehren, können die Bürstenmittel (21 und 22) schließlich ihre eigene Drehrichtung umkehren und dass das mögliche Rückhaltemittel (29), das an der Vorderseite in Bezug auf die Bürstenmittel (21 und 22) und in Bezug auf die Vorschubrichtung der Vorrichtung (10) angeordnet ist, von der begehbaren Oberfläche (11) angehoben wird.

25. Vorrichtung (10) nach Anspruch 1, **dadurch gekennzeichnet, dass** die kastenförmige Struktur des verlängerten Körpers (56) innen mit einem verlängerten Befestigungsmittel (66) versehen ist, das vertikal von einer angehobenen Ruheposition in eine abgesenkte Betriebsposition und umgekehrt gleiten kann, um die Vorrichtung (10) in ihrer Ausgangsposition zu verriegeln oder zu entriegeln.

26. Vorrichtung (10) nach Anspruch 25, **dadurch gekennzeichnet, dass** das verlängerte Befestigungsmittel (66) aus einer vertikalen Stange (71) besteht, an der eine horizontale Stange (68) an ihrem oberen Teil befestigt ist und gleitend in einem vertikalen hohlen Führungselement (67) aufgenommen ist, und durch den Bediener mittels der horizontalen Stange (68) bewegt wird, die mit mindestens einem ihrer seitlichen Endabschnitte über die vertikalen Seitenwände des Körpers (56) hinaus verlängert ist, mittels zweier vertikaler durchgehender Schlitze (70), die an diesem letzteren vorgesehen sind und zueinander spiegelbildlich sind, wobei das verlängerte Befestigungsmittel (66) in seiner angehobenen Ruheposition mittels Befestigungsmitteln des magnetischen Typs oder mittels einer Klinke, in die die horizontalen Stange (68) in Eingriff steht, gehalten wird, wobei das verlängerte Befestigungsmittel (66) in seiner abgesenkten Betriebsposition mit seinem unteren Endabschnitt (73) in ein Hubendelement (72) in Eingriff steht, das innen des Bodens des Verbindungselements (14) positioniert ist.

27. Vorrichtung (10) nach Anspruch 26, **dadurch gekennzeichnet, dass** eine Mehrzahl von Rädern (74) unter dem Rahmen (16) erhöht befestigt ist, vorzugsweise vorne in Bezug auf die Bürstenmitteln (21 und 22), so dass, wenn sich die Befestigungsmittel (66) in ihrer Betriebsposition befinden, der Bediener die Vorrichtung (10) neigen kann, indem er diese Räder (74) an der begehbaren Oberfläche (11) anliegt, so dass es möglich ist, sie mit den Bürsten (21 und 22) und das Rückhaltemittel (29), die sich in einer angehobenen Position befinden, leicht zu transportieren.

28. Vorrichtung (10) nach einem der vorhergehenden Ansprüche, **dadurch gekennzeichnet, dass** der verlängerte Körper (56) mit seinem unteren Endabschnitt (58) mit dem oberen Endabschnitt des Verbindungselements (14) mittels eines Verbindungsmittels (75) verbunden ist, die in ihrer Position verriegelbar ist und eingerichtet ist, das Steuermittel (13) in einer Betriebsposition zu halten oder es aus einer solchen Position freizugeben, um ihm zu erlauben, geneigt zu werden, ohne in das Verbindungselement (14) einzugreifen, um die Gesamtabmessungen zu verringern und es zu verpacken oder zu lagern, wobei ein solches Verbindungsmittel (75) aus einem unteren Teil (76) besteht, der kippbar mit einem oberen Teil (77) verbunden ist, der in einer anliegenden Position übereinander mittels eines Verriegelungsmittels (78), das beispielsweise aus einem Bolzen besteht, verriegelt werden kann.

29. Vorrichtung (10) nach Anspruch 28, **dadurch gekennzeichnet, dass** die Teile (76 und 77) jeweils mit einer vertikalen konzentrischen Durchgangsbohrung (79, 80) versehen sind, das eingerichtet ist, den Durchgang der vertikalen Stange (71) des Befestigungsmittels (66) zu ermöglichen.

30. Vorrichtung (10) nach einem der vorhergehenden Ansprüche, **dadurch gekennzeichnet, dass** der verlängerte Körper (56) mit einem äußeren Sitz entlang einer vertikalen Vorderwand oder Rückwand desselben versehen ist, der eingerichtet ist, ein Mittel zur Energieversorgung (57), wie beispielweise eine wiederaufladbare Batterie, die eingerichtet ist, alle elektrischen und elektronischen Bauteile der Vorrichtung (10) mit Energie zu versorgen, abnehmbar aufzunehmen.

## Revendications

1. Dispositif (10) réalisé en tant que machine autolaveuse pour le nettoyage de surfaces praticables (11), comme par exemple les sols, les trottoirs, les routes asphaltées, etc. pour le nettoyage de surfaces praticables, adapté pour avancer automatiquement et être dirigé manuellement par un opérateur, et constitué par un corps de base inférieur de nettoyage (12) relié sur sa partie supérieure au moyen d'un élément d'articulation mobile (14) à un moyen de direction allongé vers le haut (13), ledit corps de base inférieur (12) comprenant un cadre (16) auquel sont fixés un ou plusieurs moyens de brossage inférieurs (21, 22) et actionnés en rotation au moyen de moyens d'actionnement électriques respectifs (27, 28), et adaptés pour brosser les surfaces praticables (11), en enlevant la saleté et pour créer une friction avec la surface praticable (11) de manière à faire avancer automatiquement de façon rectiligne le dispositif (10), au moins un système de mouillage fixé au cadre (16), et positionné en correspondance desdits moyens de brossage (21, 22) et adapté pour les mouiller, et au moins un système de collecte de la saleté fixé au cadre (16), positionné à l'arrière desdits moyens de brossage (21, 22) par rapport à la direction de déplacement du dispositif (10) et adapté pour collecter la saleté brossée par les mêmes moyens de brossage, ledit système de mouillage étant constitué par un moyen de confinement (38), adapté pour contenir le liquide de nettoyage, d'un moyen de pompage électrique (39), adapté pour aspirer le liquide de nettoyage, et d'un moyen de distribution (40) pour distribuer un tel liquide, ledit système de collecte de saleté étant constitué par au moins un moyen de retenue (29), adapté pour retenir la saleté transportée par les moyens de brossage (21, 22), au moins un moyen d'aspiration (30), adapté pour aspirer la saleté retenue, et au moins un moyen de collecte (31), adapté pour contenir la saleté aspirée, ledit moyen de direction (13) comprenant un corps allongé vers le haut (56) relié à son sommet à une poignée (60), et étant du type inclinable dans n'importe quelle direction à partir d'une position de départ perpendiculaire par rapport au corps inférieur (12) et étant adapté pour diriger le dispositif (10), **caractérisé en ce que** ledit élément de jonction (14) est constitué par un élément de poussée (14) allongé, élastique et creux à l'intérieur, contraint avec sa partie d'extrémité inférieure (54) au cadre (16), dans le point de barycentre du corps inférieur (12) et avec sa partie d'extrémité supérieure (55) au moyen de direction (13), et ce, à partir de la position de repos de celui-ci, dans laquelle il a une extension rectiligne vers le haut, il peut être incurvé vers le bas en appliquant une force de poussée vers le bas et vers l'extérieur à sa partie d'extrémité supérieure (55) et dans n'importe quelle direction par rapport à son axe vertical lorsqu'il est dans sa position de repos, ce qui permet d'incliner vers le bas le moyen de direction (13), et dans cette position incurvée pouvant tourner le long de l'axe vertical de la position de repos du même élément élastique, sans que le corps inférieur (12) soit intéressé par une telle rotation, en diminuant la force de poussée vers le bas de la partie terminale supérieure (55), l'élément élastique (14) revenant automatiquement vers sa position de repos, un tel élément élastique (14) étant également adapté pour recevoir un moment de torsion à sa partie d'extrémité supérieure (55), au moyen dudit moyen de direction (13), pour ensuite appliquer ledit moment de torsion, avec sa partie d'extrémité inférieure (54), au cadre (16) du corps inférieur (12), de sorte que ce dernier tourne sur son axe perpendiculaire dans la mesure vers le haut de l'angle désiré par l'opérateur, sans être incliné.

2. Dispositif (10) selon la revendication 1, **caractérisé en ce qu'**un tel élément de poussée (14) allongé, élastique et creux à l'intérieur est constitué par un élément à ressort, comme par exemple au moins un ressort, en métal ou en polymère.

3. Dispositif (10) selon la revendication 2, **caractérisé en ce que** ledit élément à ressort peut être constitué par au moins deux ressorts insérés ou co-moulés concentriquement l'un dans l'autre, en métal et/ou en polymère.

4. Dispositif (10) selon la revendication 2, **caractérisé en ce que** ledit élément à ressort peut être constitué par un soufflet métallique, par exemple en acier, avec un noyau en matériau élastique et semi-rigide, par exemple en caoutchouc.

5. Dispositif (10) selon la revendication 2, **caractérisé en ce que** ledit élément à ressort peut être constitué par un tube en matière élastique et semi-rigide, comme par exemple le caoutchouc, muni d'un renfort interne constitué de fibres de renforcement tissées entre elles, comme par exemple des fils en métal, nylon, Kevlar, carbone, technopolymère.

6. Dispositif (10) selon la revendication 2, **caractérisé en ce que** ledit élément à ressort peut être constitué par un ensemble de ressorts à disque conique reliés entre eux par leurs bords, disposés alternativement à la suite les uns des autres, par conséquent, la grande base du premier ressort à disque conique est reliée à la grande base d'un deuxième ressort à disque conique suivant, et la base serrée de ce dernier est reliée à la base serrée d'un troisième ressort à disque conique suivant, et ainsi de suite pour les autres ressorts à disque conique qui composent l'ensemble de l'élément élastique allongé (14).

7. Dispositif (10) selon la revendication 1, **caractérisé en ce que** lesdits moyens d'actionnement électriques (27, 28), constitués par exemple par des moteurs électriques, sont disposés sur la partie supérieure dudit cadre (16), et au-dessus des moyens de brossage respectifs (21 et 22), qu'ils doivent actionner, lesdits moyens de brossage (21, 22) étant disposés les uns à côté des autres de manière transversale par rapport à l'orientation du corps inférieur (12), et tournant en sens inverse les uns des autres selon un axe légèrement incliné vers l'intérieur par rapport à la verticale du dispositif (10) penché sur un plan horizontal.

8. Dispositif (10) selon la revendication 1, **caractérisé en ce que** ledit moyen de direction (13) comprend à son sommet un panneau de commande et de réglage (61) associé à au moins un premier moyen électronique de contrôle et de commande pour activer le dispositif (10) et pour sélectionner un programme opérationnel, chargé à l'avance dans le premier moyen électronique de contrôle et de commande, des éléments constitutifs électriques et électroniques du même dispositif (10), ledit panneau de commande et de réglage (61) étant constitué au moins par un écran, où les données du dispositif (10) sont visibles, et par une pluralité de moyens de réglage, comme par exemple des boutons-poussoirs et/ou des boutons et/ou des commandes d'écran tactile, qui sont intégrés dans l'écran susmentionné.

9. Dispositif (10) selon la revendication 8, **caractérisé en ce que** ledit premier moyen de contrôle et de commande est associé à au moins un second moyen électronique de contrôle et de commande, constitué par exemple par une carte électronique équipée d'au moins un microprocesseur également, et positionné sur le cadre (16), ce second moyen de contrôle et de commande étant adapté pour recevoir des instructions du premier moyen de contrôle et de commande et régler l'activation et l'extinction des moyens électriques et électroniques compris dans le dispositif (10) et exécuter le programme opérationnel sélectionné et régler l'intensité de fonctionnement.

10. Dispositif (10) selon la revendication 1, **caractérisé en ce que** ladite poignée (60) est du type anneau fermé et se prolonge vers le haut par deux premiers segments allongés (62) identiques et spéculaires l'un par rapport à l'autre et légèrement inclinés vers l'extérieur, et se poursuit par deux seconds segments allongés (63) identiques et spéculaires l'un par rapport à l'autre et étendus vers l'avant, et se referme par un segment allongé horizontal (64) qui relie la partie d'extrémité supérieure des deux seconds segments (63).

11. Dispositif (10) selon la revendication 1, **caractérisé en ce que** ledit moyen de retenue (29) pour la saleté est constitué par un support semi-circulaire allongé (29') pourvu d'au moins un trou vertical traversant (32) et qui supporte sur son bord inférieur au moins un élément allongé (33), fait d'un matériau semi-rigide, comme par exemple le caoutchouc, ledit support (29') étant positionné transversalement par rapport à l'orientation de l'appareil (10), avec sa partie concave (34) tournée vers les deux moyens de brossage (21 et 22), et étant relié à la partie inférieure du cadre (16) au moyen d'au moins deux paires de leviers (35, 36), pivotant à leurs parties d'extrémité (37) parallèles l'une à l'autre, et disposés longitudinalement par rapport à l'orientation du dispositif (10), ces leviers (35, 36) étant adaptés pour tourner sur leurs parties d'extrémité (37) en fonction du mouvement vertical provoqué par le moyen de retenue (29) qui suit la conformation de la surface praticable (11), ou en étant soulevé ou abaissé manuellement ou automatiquement lorsque l'opérateur le juge nécessaire, ledit moyen de retenue (29) étant maintenu soulevé au moyen de moyens de fixation rapide ou de moyens magnétiques ou électro-magnétiques, ou équivalents.

12. Dispositif (10) selon la revendication 11, **caractérisé en ce que** lesdits leviers (35, 36) sont en métal élastique, de préférence en acier harmonique, et sont structurés et adaptés pour être pliés transversalement lorsque le moyen de retenue (29) heurte latéralement un obstacle extérieur (80), pour revenir ensuite automatiquement dans sa position initiale après avoir franchi un tel obstacle extérieur.

13. Dispositif (10) selon la revendication 11, **caractérisé en ce que** lesdits leviers (35, 36) peuvent être tournés verticalement au moyen d'un moyen d'actionnement électrique (51), placé sur la partie supérieure du cadre (16).

14. Dispositif (10) selon la revendication 1, **caractérisé en ce que** lesdits moyens de retenue (29) sont pourvus de deux roues de support (42), placées latéralement et/ou centralement par rapport au support allongé (29') et sont fixées au moyen de supports métalliques (43).

15. Dispositif (10) selon la revendication 11, **caractérisé en ce que** ledit moyen d'aspiration (30) est du type électrique, et est disposé sur la partie supérieure dudit cadre (16) et au-dessus desdits moyens de retenue (29), et avec son embouchure d'entrée est relié de manière communicante audit trou de passage vertical (32), et avec son embouchure de sortie est relié de manière communicante audit moyen de collecte (31).

16. Dispositif (10) selon la revendication 15, **caractérisé en ce que** ledit moyen de collecte (31) est constitué par un premier réservoir disposé de manière amovible au-dessus de la demi-partie arrière dudit cadre (16) et des composants placés dans cette partie de cadre, et présente une base qui suit le contour et les dimensions de la partie de cadre sur laquelle il s'appuie et une hauteur qui n'interfère pas avec l'élément de jonction (14).

17. Dispositif (10) selon la revendication 1, **caractérisé en ce que** ledit moyen de pompage électrique (39), constitué par exemple par une pompe électrique, est placé sur le cadre (16), dans la zone centrale de ce dernier, et est associé de manière communicante avec ledit moyen de confinement (38), qui est constitué par un second réservoir disposé de manière amovible au-dessus de la demi-partie avant dudit cadre (16) et des composants placés dans cette partie de cadre, et qui a une base qui suit le contour et les dimensions de la partie de cadre sur laquelle il est appuyé et une hauteur qui n'interfère pas avec l'élément de jonction (14).

18. Dispositif (10) selon les revendications 15 et 17, **caractérisé en ce que** le premier réservoir (31) et le second réservoir (38) sont joints l'un à l'autre et forment un corps unique (45) ayant une surface supérieure (46) presque continue et inclinée frontalement vers le bas et des parois latérales (47) inclinées vers l'intérieur et vers le haut, et ayant enfin un trou vertical (41), en correspondance de l'élément de jonction (14), de telle sorte que ce dernier puisse y être inséré et être libre d'exercer sa fonction.

19. Dispositif (10) selon la revendication 18, **caractérisé en ce que** ledit corps (45), le long de son contour de base inférieur, est également pourvu d'une pluralité d'évidements de forme (48) identiques et équidistants les uns des autres, en correspondance de chacun desquels, au-dessus dudit cadre (16), un moyen résistant aux chocs (49) respectif est logé et fait partiellement saillie vers l'extérieur du dispositif (10), un tel moyen résistant aux chocs (49) est constitué par exemple d'un rouleau pivotant avec un axe de rotation vertical, libre en rotation, et fait d'un matériau élastique semi-rigide, comme par exemple le caoutchouc.

20. Dispositif (10) selon l'une des revendications précédentes, **caractérisé en ce qu'au** moins un moyen d'assainissement électrique (52) est fixé à la partie inférieure du cadre (16), à l'arrière dudit moyen de retenue (29) par rapport à la direction d'avancement dudit dispositif (10), un tel moyen d'assainissement (52) étant constitué comme par exemple par une ou plusieurs lampes UV et étant adapté pour compléter l'assainissement de la surface praticable (11) qui vient d'être traitée, et au moins une roue pivotante (79) étant positionnée dans la partie inférieure du cadre (16), à l'arrière par rapport audit moyen d'assainissement électrique (52), et étant adaptée pour supporter la partie arrière du corps inférieur (12), en évitant que ce dernier soit incliné vers l'arrière.

21. Dispositif (10) selon la revendication 8, **caractérisé en ce que** ledit premier moyen électronique de contrôle et de commande est également associé à au moins un premier moyen de détection supplémentaire, positionné sur la partie supérieure du moyen de direction (13) et adapté pour détecter la présence de l'opérateur à proximité du dispositif (10), de telle sorte que si le dispositif (10) est en état de fonctionnement et que l'opérateur n'est pas détecté, ledit premier moyen électronique de contrôle et de commande envoie l'ordre au second moyen électronique de contrôle et de commande de désactiver tous les moyens électriques et électroniques qui sont opérationnels à ce moment-là, pour les réactiver ensuite lorsque l'opérateur sera détecté.

22. Dispositif (10) selon la revendication 21, **caractérisé en ce que** ledit premier moyen de détection est constitué par un capteur de proximité du type à ultrasons ou du type optique et est positionné dans la zone du panneau de commande et de réglage (61).

23. Dispositif (10) selon la revendication 21, **caractérisé en ce que** ledit premier moyen de détection est constitué par un capteur de proximité de type capacitif et positionné sur la poignée (60).

24. Dispositif (10) selon l'une des revendications précédentes, **caractérisé en ce que** ledit premier moyen de contrôle et de commande est également associé au moyen capteur (53), constitué par exemple par un capteur de type gyroscope ou de type inertiel, positionné sur le cadre (16), et adapté pour détecter la direction d'avancement du dispositif (10), c'est-à-dire que si l'opérateur pousse ou tire le même dispositif (10) pour en inverser la direction d'avancement, les moyens de brossage (21 et 22) peuvent éventuellement inverser leur propre sens de rotation et les éventuels moyens de retenue (29) positionnés à l'avant par rapport aux moyens de brossage (21 et 22) par rapport à la direction d'avancement du dispositif (10) peuvent être soulevés de la surface praticable (11).

25. Dispositif (10) selon la revendication 1, **caractérisé en ce que** ladite structure en forme de boite du corps allongé (56) est pourvue à l'intérieur d'un moyen de fixation allongé (66), qui peut glisser verticalement d'une position de repos élevée à une position opérationnelle abaissée, et vice versa, pour verrouiller ou déverrouiller le dispositif (10) dans sa position de départ.

26. Dispositif (10) selon la revendication 25, **caractérisé en ce que** ledit moyen de fixation allongé (66) est constitué par une tige verticale (71) à laquelle est fixée une tige horizontale (68) sur sa partie supérieure et logée de manière coulissante dans un élément de guidage creux vertical (67), et déplacée par l'opérateur au moyen de la tige horizontale (68), qui est prolongée avec au moins une de ses parties d'extrémité latérale au-delà des parois verticales latérales du corps (56), au moyen de deux fentes verticales traversantes (70) prévues sur ce dernier et spéculaires l'une par rapport à l'autre, ledit moyen de fixation allongé (66) étant maintenu dans sa position de repos relevée au moyen de moyens de fixation du type magnétique ou au moyen d'un cliquet dans lequel est engagée la tige horizontale (68), ledit moyen de fixation allongé (66), dans sa position abaissée opérationnelle, est engagé avec sa partie d'extrémité inférieure (73) dans un élément de fin de course (72), positionné à l'intérieur de la base de l'élément de jonction (14).

27. Dispositif (10) selon la revendication 26, **caractérisé en ce qu'**une pluralité de roues (74) est fixée sous le cadre (16) de manière surélevée, de préférence à l'avant par rapport aux moyens de brossage (21 et 22), de telle sorte que lorsque le moyen de fixation (66) est dans sa position opérationnelle, l'opérateur peut incliner le dispositif (10) en appuyant ces roues (74) sur la surface praticable (11), ce qui permet de le transporter facilement avec les brosses (21 et 22) et les moyens de retenue (29) qui sont en position relevée.

28. Dispositif (10) selon l'une des revendications précédentes, **caractérisé en ce que** ledit corps allongé (56) est relié avec sa partie d'extrémité inférieure (58) à la partie d'extrémité supérieure de l'élément de jonction (14) au moyen d'un moyen de jonction (75), verrouillable en position et adapté pour maintenir le moyen de direction (13) dans une position opérationnelle de celui-ci ou pour le libérer d'une telle position pour lui permettre d'être incliné sans intervenir sur l'élément de jonction (14), de manière à en réduire l'encombrement et l'emballage ou le stockage, un tel moyen de jonction (75) étant constitué par une partie inférieure (76) reliée de manière basculante à une partie supérieure (77), cette dernière pouvant être verrouillée en position adjacente, l'une au-dessus de l'autre, au moyen d'un moyen de verrouillage (78), constitué par exemple d'un boulon.

29. Dispositif (10) selon la revendication 28, **caractérisé en ce que** lesdites parties (76 et 77) sont pourvues chacune d'un trou vertical concentrique traversant respectif (79, 80), adapté pour permettre le passage de la tige verticale (71) du moyen de fixation (66).

30. Dispositif (10) selon l'une des revendications précédentes, **caractérisé en ce que** ledit corps allongé (56) est pourvu d'une assise externe le long d'une de ses parois verticales avant ou arrière, adaptée pour loger de manière amovible un moyen d'alimentation en énergie électrique (57), comme par exemple une batterie rechargeable, adaptée pour alimenter tous les composants électriques et électroniques du dispositif (10).
